# EUROPEAN PATENT APPLICATION

(11) **EP 2 236 157 A1**
(43) Date of publication of application: **06.10.2010**
(21) Application number: 09156755.2
(22) Date of filing: 30.03.2009
(51) Int. Cl.: A61K 45/06

(54) **Pipamperone and a second agent in treating mood and anxiety disorders**

(71) Applicant: PharmaNeuroBoost N.V., 3570 Alken (BE)
(72) Inventor: Buntinx, Erik, 3570, Alken (BE); van der Geest, Ronald, 4835 AA, Breda (NL)
(74) Representative: De Clercq, Ann G. Y.

(57) **Abstract**

The present invention relates to the use of combinations comprising pipamperone and an SSRI, SNDRI or SNRI and compositions comprising the same for the treatment of mood or anxiety disorders.

## Description

### Field of the invention

The invention relates to the field of neuropsychiatry. More specifically, the invention relates to the use of pipamperone in augmenting and in a faster onset of serotonin re-uptake inhibitors, such as SSRIs, SNDRIs and SNRIs, in treating mood and anxiety disorders.

### Background of the invention

Conventionally, mental disorders are divided into types based on criteria sets with defining features.

DSM-IV (American Psychiatric Association, (1993 - ISBN 0 - 89042 - 061 - 0)) is the in the art well-known gold standard of such a categorical classification. In DSM-IV, there is no assumption that each category of mental disorder is a completely discrete entity with absolute boundaries dividing it from other mental disorders or from no mental disorder. There is also no assumption that all individuals described as having the same mental disorder are alike in all important ways. Individuals sharing a diagnosis are likely to be heterogeneous even in regard to the defining features of the diagnosis. Thus, the categorical defined mental disorders mood and anxiety disorders are having an external and even internal variable co-incidence of symptoms concerning mood and anxiety.

In a dimensional system, clinical presentations are classified based on quantification of attributes, i.e. dysfunctions rather than the assignment to categories. This works best in describing phenomena, which are distributed continuously and which do not have clear boundaries. Emotion dysregulation is known as such an attribution or dysfunction that plays an important role in the development and course of mental disorders *(*Gross, J. J. & Munoz, R. F., 1995, Emotion regulation and mental health, Clinical Psychology: Science and Practice, 2, 151-164*;* Mennin, D.S., Heimberg, R. G., Turk, C. L. & Fresco, D. M., 2002, Applying an emotion regulation framework to integrative approaches to generalized anxiety disorder, Clinical Psychology: Science and Practice, 9, 85-90*;* Linehan, M. M., 1993, Cognitive-behavioral treatment of borderline personality disorder, New York, The Guilford Press*;* Gratz, K. L., Roemer, L., 2001 & 2004, Multidimensional assessment of emotion regulation and dysregulation: development, factor structure, and initial validation of the Difficulties in Emotion Regulation Scale, Annual meeting of the Association for Advancement of Behavior Therapy, Nov. 2001 & Journal of Psychopathology and Behavioral Assessment, Vol. 26, No. 1, March 2004*)* besides behavioural and cognitive dysfunctions. Finally, in the biological system, mental disorders are defined on other levels of abstraction than in the categorical and dimensional system. Structural pathology (e.g. amyloid plaques in Alzheimer Disease), etiology (e.g. HIV Dementia) and deviance from a physiological norm (e.g. reduced cerebral blood flow) are often used as indicative biological markers for a mental disorder. The underlying dysregulation of various neurotransmittor systems (glutaminergic, GABAergic, cholinergic, monoaminergic (noradrenergic, dopaminergic, serotonergic), etc.) is the in the art used model for the explanation of the biological determinants of the clinical presentation of mental disturbances.

Mood disorders include major depression, bipolar disorder (combining episodes of both mania and depression) and dysthymia. As a group, mood disorders are one of the most common mental illnesses in the general population. Approximately 8% of adults will experience major depression at some time in their lives, while approximately 1% will experience bipolar disorder. Other studies have reported that between 3% and 6% of adults will experience dysthymia during their lifetime, and that between 0.6% and 1% of adults will have a manic episode during their lifetime. Because of their high prevalence, economic cost, risk of suicide and loss of quality of life, mood disorders present a serious public health concern in society. Also, depression and mania cause significant distress and impairment in social, occupational, educational or other important areas of functioning. According to the World Health Organization (WHO), major depression is the fourth leading cause of disability adjusted life years (DALYs) in the world. Major depression is the leading cause of years of life lived with disability (YLD) and bipolar is the sixth leading cause. Social and economic effects of mood disorders include functional impairment, disability or lost work productivity, and increased use of health services.

Depression is a serious mood disorder which affects millions of people, while the number of people being diagnosed with depression has increased dramatically. There is no single known cause of depression. Rather, it likely results from a combination of genetic, biochemical, environmental, and psychological factors. Nevertheless, important neurotransmitters appear to be out of balance. In particular, serotonin signaling is affected in depressed patients. Hence, depression is treated with antidepressants which work to normalize neurotransmitters, notably serotonin and norepinephrine. Other antidepressants work on the neurotransmitter dopamine. Although it is found that these particular neurotransmitters are involved in regulating mood, it is uncertain of the exact ways in which they work.

Anxiety disorders are the most common of emotional disorders and affect more than 25 million Americans. Anxiety disorder is a persistent fear of social or performance situations that might involve exposure to unfamiliar people or possible scrutiny by others. Many forms and symptoms may include: overwhelming feelings of panic and fear, uncontrollable obsessive thoughts, painful, intrusive memories, recurring nightmares, and even physical symptoms such as feeling sick to your stomach, "butterflies" in your stomach, heart pounding, startling easily, and muscle tension. This condition, which often remains undetected and untreated, undermines a person's ability to become self-sufficient and impedes efforts to reduce welfare costs through return-to-work programs. Patients with this disorder commonly underperform educationally, have a lower probability of marrying, a lower economic status, and a higher probability of losing their job. The early onset of symptoms in adolescence interferes with the acquisition of social skills, resulting in social isolation. Patients with anxiety disorders are frequent users of the public health system. All these problems can be worsened if the anxiety disorder is accompanied by other mental disorders. Nevertheless, anxiety disorder is commonly under-diagnosed. The limitation of lives and the economic and social problems are always underestimated. An early diagnosis and treatment are key elements for lowering the social and economic burden of social anxiety disorder. Types of Anxiety Disorders include panic disorder, phobias, obsessive-compulsive disorer (OCD), posttraumatic stress disorder (PTSD) and generalized anxiety disorder (GAD). If left untreated, anxiety disorders can have severe consequences resulting in avoidance behavior which may create problems by conflicting with job requirements, family obligations or other basic activities of daily living. Moreover, many people who suffer from an untreated anxiety disorder are prone to other psychological disorders, such as depression, and they have a greater tendency to abuse alcohol and other drugs. Their relationships with family members, friends and coworkers may become very strained, while their job performance may falter.

Obsessions are upsetting and irrational thoughts which keep reoccurring. They cause great anxiety, which cannot be controlled through reasoning. Common obsessions include preoccupations with dirt or germs, nagging doubts, and a need to have things in a very particular order. To minimize these obsessions, many people with obsessive-compulsive disorder (OCD) engage in repeated behaviour, or compulsions. Examples include repeated hand washing, constant rechecking to satisfy doubts, and following rigid rules of order. Compulsive behaviour can be very disruptive to normal daily routines and social relationships.

Hence, because of their high prevalence, apart from the impact on the individual itself and his relation, mood and anxiety disorders have a major effect on economy. This effect is dual in nature - first, with the associated loss of productivity in the workplace due to absenteeism and diminished effectiveness; and second, with the high health care costs attributable to primary care visits, hospitalizations and medication. At the individual and family level, the loss of income and cost of medication create a strain on the family financial resources. Hence, mood and anxiety disorders have a major economic impact through associated health care costs as well as lost work productivity. Accordingly, there is a substantial need to diagnose and treat these disorders.

Mainstream treatment for mood and anxiety disorders consists of the prescription of antidepressants (for both mood and anxiety disorders) and anxiolytic agents (mainly for anxiety disorders).

The newest and most popular types of antidepressant medications are called selective serotonin reuptake inhibitors (SSRIs). SSRIs include fluoxetine (Prozac), citalopram (Celexa), sertraline (Zoloft) and several others. Serotonin and norepinephrine reuptake inhibitors (SNRIs) are similar to SSRIs and include venlafaxine (Effexor) and duloxetine (Cymbalta). SNDRIs are so-called triple reuptake inhibitors, which elevate extracellular plasma concentrations of all three monoamine neurotransmitters, serotonin, dopamine and norepinephrine in the synaptic cleft. No SNDRIs are yet on the market, although one of these agents GlaxoSmithKline's NS2359 is currently in clinical trials, and other compounds such as brasofensine and tesofensine are under development.

For all classes of antidepressants, patients must take regular doses for at least three to four weeks before they are likely to experience a full therapeutic effect.

Apart from the delayed effectiveness, antidepressants are also known to cause side effects. The most common side effects associated with SSRIs and SNRIs include: headache, nausea, insomnia, nervousness, agitation and sexual problems. For instance, citalopram is a commonly used SSRI. Citalopram can have a number of adverse effects. In clinical trials, over 10% of patients reported one or more of the following side effects: fatigue, drowsiness, dry mouth, increased sweating (hyperhidrosis), trembling, headache, dizziness, sleep disturbances, insomnia, cardiac arrhythmia, hallucinations, blood pressure changes, nausea and/or vomiting, diarrhea, heightened anorgasmia in females, impotence and ejaculatory problems in males. In some cases, allergic reactions, convulsions, mood swings, anxiety and confusion have been reported. Also sedation may be present during treatment of antidepressants, for instance citalopram.

Benzodiazepines are prescribed for short-term relief of severe and disabling anxiety. Common medications are lorazepam (Ativan), clonazepam (Klonopin), alprazolam (Xanax), and diazepam (Valium). Benzodiazepines may also be indicated to cover the latent periods associated with the medications prescribed to treat an underlying anxiety disorder. There is a risk of a benzodiazepine withdrawal and rebound syndrome after continuous usage past two weeks. There is also the added problem of the accumulation of drug metabolites and adverse effects. At higher dosage hypnotic properties occur. A further anxiolytic agent is buspirone, which is serotonin 1A agonist. Although it lacks the sedation and the dependence associated with benzodiazepines, it is less effective than benzodiazepines in patients who have been previously treated with benzodiazepines as the medication does not provide the sedation that these patients may expect or equate with anxiety relief.

Apart from the side-effects, clinical or real effectiveness of psychopharmaca is very rare via common pooping-out; many treatment-refractory patients and up to half of patients fail to attain remission *(*S. M. Stahl, Essential Psychopharmacology, Depression and Bipolar Disorders, 151, University Press; 2 edition (June 15, 2000); ISBN: 0521646154*).* Implications of not attaining remission for Mental Disorders are increased relapse rates, continuing functional impairment and increased suicide rate *(S. M. Stahl, ibid).* Clinical causes of not attaining remission by the Current Psychopharmacological Compounds are inadequate early treatment, underlying emotion dysregulation (affecting instability - hypersensitivity - hyperaesthesia - dissociative phenomena, etc.) and competitive antagonism.

Hence, there is a need for more efficient, selective and efficacious medicaments for treating mood and anxiety disorders.

WO 2005/053796 relates to low doses of antagonists of 5-HT2A and D4 receptors in order to augment the effects of these compounds in mental disorders. One of these antagonists is pipamperone, which is active in the range of 5-15 mg per day for a broad class of compounds. WO 2005/053796 is silent on other dose ranges of pipamperone.

The instruction leaflet from the manufacturer Janssen Cilag B.V details psychoses and the symptomatic treatment of serious forms of agitation and anxiety as the therapeutic indications for pipamperone. The leaflet warns against the use of pipamperone in depression. The recommended initial dose is 40 to 80 mg pipamperone a day. If necessary, it is recommended to increase the dose up to a maximum of 360 mg pipamperone per day.

The present inventor surprisingly found that the effect of serotonin re-uptake inhibitors, including SSRIs, SNDRIs and SNRIs, may be further ameliorated at a dose of more than 15 to about 20 mg pipamperone per day.

### Summary of the invention

(1) The present invention relates to the use of pipamperone or a pharmaceutically acceptable salt thereof for the preparation of a medicament for treating a mood disorder or anxiety disorder, wherein said pipamperone or a pharmaceutically acceptable salt is to be administered to a patient in a daily dose ranging between more than 15 and about 20 mg of the active ingredient, and wherein a second compound is to be administered simultaneously with, separate from or sequential to said pipamperone to augment the therapeutic effect of said second compound or to provide a faster onset of said second compound, said second compound is selected from the group consisting of: selective serotonin, nor-adrenaline and dopamine re-uptake inhibitor (SNDRI), selective serotonin and nor-adrenaline re-uptake inhibitor (SNRI) and selective serotonin re-uptake inhibitor (SSRI).
(2) The present invention further relates to the use of pipamperone or a pharmaceutically acceptable salt thereof according to (1), wherein said pipamperone is to be administered daily at least one day before administering said second compound.
(3) The present invention further relates to the use of pipamperone or a pharmaceutically acceptable salt thereof according to (1) or (2), wherein said second compound is a selective serotonin, nor-adrenaline and dopamine re-uptake inhibitor (SNDRI) compound,
(4) preferably wherein said selective serotonin, nor-adrenaline and dopamine re-uptake inhibitor (SNDRI) compound is selected from the group consisting of NS 2330, NS2359, McN 5652, DOV 216,303 and DOV 21,947, or a pharmaceutically acceptable salt thereof.
(5) The present invention further relates to a pharmaceutical composition comprising (a) pipamperone, and (b) a selective serotonin, nor-adrenaline and dopamine re-uptake inhibitor (SNDRI) compound for treating a mood disorder or anxiety disorder, wherein said pipamperone is to be administered to a patient in a daily dose ranging between more than 15 and about 20 mg of the active ingredient, (6) preferably, wherein said selective serotonin, nor-adrenaline and dopamine re-uptake inhibitor (SNDRI) compound is selected from the group consisting of NS 2330, NS2359, McN 5652, DOV 216,303 and DOV 21,947, or a pharmaceutically acceptable salt thereof.
(7) The present invention further relates to the use of pipamperone or a pharmaceutically acceptable salt thereof according to (1) or (2), wherein said second compound is a selective serotonin and nor-adrenaline re-uptake inhibitor (SNRI) compound, (8) preferably wherein said selective serotonin and nor-adrenaline re-uptake inhibitor (SNRI) compound is selected from the group consisting of venlafaxine, tomoxetine, tandamine, talsupram, talopram, nefazodone, milnacipran, LY 113.821, duloxetine, desvenlafaxine and amoxapine, or a pharmaceutically acceptable salt thereof, (9), even more preferably, wherein said
   - venlafaxine, or a pharmaceutically acceptable salt thereof, is to be administered in a daily dose ranging between 75 and 300 mg of the active ingredient;
   - tomoxetine, or a pharmaceutically acceptable salt thereof, is to be administered in a daily dose ranging between 0.475 and 3.8 mg/kg of the active ingredient;
   - milnacipran, or a pharmaceutically acceptable salt thereof, is to be administered in a daily dose ranging between 50 and 200 mg of the active ingredient;
   - duloxetine, or a pharmaceutically acceptable salt thereof, is to be administered in a daily dose ranging between 40 and 120 mg of the active ingredient;
   - nefazodone, or a pharmaceutically acceptable salt thereof, is to be administered in a daily dose ranging between 100 and 600 mg of the active ingredient;
   - amoxapine, or a pharmaceutically acceptable salt thereof, is to be administered in a daily dose ranging between 100 and 600 mg of the active ingredient; and/or
   - desvenlafaxine, or a pharmaceutically acceptable salt thereof, is to be administered in a daily dose ranging between 50 and 250 mg of the active ingredient.
(10) The present invention further relates to a pharmaceutical composition comprising (a) pipamperone and (b) a selective serotonin and nor-adrenaline re-uptake inhibitor (SNRI) compound as a combined preparation for treating a mood disorder or anxiety disorder, wherein said pipamperone is to be administered to a patient in a daily dose ranging between more than 15 and about 20 mg of the active ingredient (11) preferably, wherein said selective serotonin and nor-adrenaline re-uptake inhibitor (SNRI) compound is selected from the group consisting of venlafaxine, tomoxetine, tandamine, talsupram, talopram, nefazodone, milnacipran, LY 113.821, duloxetine, desvenlafaxine and amoxapine, or a pharmaceutically acceptable salt thereof, (12) even more preferably, wherein said
   - venlafaxine, or a pharmaceutically acceptable salt thereof, provided in a unitary dose of between 75 and 300 mg of the active ingredient;
   - tomoxetine, or a pharmaceutically acceptable salt thereof, is provided in a unitary dose of between 0.475 and 3.8 mg/kg of the active ingredient;
   - milnacipran, or a pharmaceutically acceptable salt thereof, is provided in a unitary dose of between 50 and 200 mg of the active ingredient;
   - duloxetine, or a pharmaceutically acceptable salt thereof, is provided in a unitary dose of between 40 and 120 mg of the active ingredient;
   - nefazodone, or a pharmaceutically acceptable salt thereof, is provided in a unitary dose of 100 and 600 mg of the active ingredient;
   - amoxapine, or a pharmaceutically acceptable salt thereof, is provided in a unitary dose of 100 and 600 mg of the active ingredient; and/or
   - desvenlafaxine, or a pharmaceutically acceptable salt thereof, is provided in a unitary dose of between 50 and 250 mg of the active ingredient.
(13) The present invention further relates to the use of pipamperone or a pharmaceutically acceptable salt thereof according to (1) or (2), wherein said second compound is a selective serotonin re-uptake inhibitor (SSRI) compound, (14) preferably said selective serotonin re-uptake inhibitor (SSRI) compound is selected from the group consisting of YM 992, VPI-013 (OPC-14523), sertraline, paroxetine, LY 214.281, LU AA 21-004, Lu 35-138, litoxetine, ifoxetine, fluvoxamine (controlled release formulation), fluvoxamine, fluoxetine, femoxetine, escitalopram, EMD 68843, cyanodothepine, citalopram, venlafaxine, milnacipran, duloxetine, desvenlafaxine cericlamine and ademethionine, or a pharmaceutically acceptable salt thereof, (15) even more preferably, wherein said:
   - fluvoxamine (controlled release formulation), or a pharmaceutically acceptable salt thereof, is to be administered in a daily dose ranging between 100 and 300 mg of the active ingredient;
   - escitalopram, and is to be administered in a daily dose ranging between 10 and 50 mg of the active ingredient;
   - citalopram, and is to be administered in a daily dose ranging between 10 and 60 mg of the active ingredient;
   - paroxetine and is to be administered in a daily dose ranging between 20 and 75 mg of the active ingredient;
   - fluoxetine and is to be administered in a daily dose ranging between 20 and 60 mg of the active ingredient;
   - fluvoxamine and is to be administered in a daily dose ranging between 50 and 300 mg of the active ingredient;
   - sertraline and is to be administered in a daily dose ranging between 25 and 300 mg of the active ingredient;
   - milnacipran and is to be administered in a daily dose ranging between 50 and 200 mg of the active ingredient;
   - venlafaxine and is to be administered in a daily dose ranging between 75 and 300 mg of the active ingredient;
   - duloxetine and is to be administered in a daily dose ranging between 40 and 120 mg of the active ingredient; and/or
   - desvenlafaxine and is to be administered in a daily dose ranging between 50 and 250 mg of the active ingredient.
(16) The present invention further relates to a pharmaceutical composition comprising (a) pipamperone and (b) a selective serotonin re-uptake inhibitor (SSRI) compound as a combined preparation for treating a mood disorder or anxiety disorder, wherein said pipamperone is to be administered to a patient in a daily dose ranging between more than 15 and about 20 mg of the active ingredient, (17) preferably, wherein said selective serotonin re-uptake inhibitor (SSRI) compound is selected from the group consisting of YM 992, VPI-013 (also known as OPC-14523), sertraline, paroxetine, LY 214.281, LU AA 21-004, Lu 35-138, litoxetine, ifoxetine, fluvoxamine (controlled release formulation), fluvoxamine, fluoxetine, femoxetine, escitalopram, EMD 68843, cyanodothepine, citalopram, venlafaxine, milnacipran, duloxetine, desvenlafaxine, cericlamine and ademethionine (preferably s-adenosylmethionine), or a pharmaceutically acceptable salt thereof, (18) even more preferably, wherein said
   - fluvoxamine (controlled release formulation), or a pharmaceutically acceptable salt thereof, is provided in a unitary dose of between 100 and 300 mg of the active ingredient;
   - escitalopram, or a pharmaceutically acceptable salt thereof, is provided in a unitary dose of between 10 and 50 mg of the active ingredient;
   - citalopram, or a pharmaceutically acceptable salt thereof, is provided in a unitary dose of between 10 and 60 mg of the active ingredient;
   - paroxetine, or a pharmaceutically acceptable salt thereof, is provided in a unitary dose of between 20 and 75 mg of the active ingredient;
   - fluoxetine, or a pharmaceutically acceptable salt thereof, is provided in a unitary dose of between 20 and 60 mg of the active ingredient;
   - fluvoxamine, or a pharmaceutically acceptable salt thereof, is provided in a unitary dose of between 50 and 300 mg of the active ingredient;
   - sertraline, or a pharmaceutically acceptable salt thereof, is provided in a unitary dose of between 25 and 300 mg of the active ingredient;
   - milnacipran, or a pharmaceutically acceptable salt thereof, is provided in a unitary dose of between 50 and 200 mg of the active ingredient;
   - venlafaxine, or a pharmaceutically acceptable salt thereof, is provided in a unitary dose of between 75 and 300 mg of the active ingredient;
   - duloxetine, or a pharmaceutically acceptable salt thereof, is provided in a unitary dose of between 40 and 120 mg of the active ingredient; and/or
   - desvenlafaxine, or a pharmaceutically acceptable salt thereof, is provided in a unitary dose of between 50 and 250 mg of the active ingredient.
(19) The present invention further relates to a pharmaceutical composition comprising (a) pipamperone and (b) selective serotonin, nor-adrenaline and dopamine re-uptake inhibitor (SNDRI), selective serotonin and nor-adrenaline re-uptake inhibitor (SNRI) or selective serotonin re-uptake inhibitor (SSRI) as a combined preparation for simultaneous, separate or sequential use for treating a mood disorder or anxiety disorder, said pipamperone is to be administered to a patient in a daily dose of between more than 15 and about 20 mg of the active ingredient.
(20) The present invention further relates to a pharmaceutical composition comprising (a) pipamperone at between more than 15 mg and about 20 mg, (b) selective serotonin, nor-adrenaline and dopamine re-uptake inhibitor (SNDRI), selective serotonin and nor-adrenaline re-uptake inhibitor (SNRI) or selective serotonin re-uptake inhibitor (SSRI), (21) preferably, wherein said SNRI is selected from the group consisting of venlafaxine, tomoxetine, tandamine, talsupram, talopram, nefazodone, milnacipran, LY 113.821, duloxetine, desvenlafaxine and amoxapine, or a pharmaceutically acceptable salt thereof, (22) preferably, wherein said SSRI is selected from the group consisting of YM 992, VPI-013 (also known as OPC-14523), sertraline, paroxetine, LY 214.281, LU AA 21-004, Lu 35-138, litoxetine, ifoxetine, fluvoxamine (controlled release formulation), fluvoxamine, fluoxetine, femoxetine, escitalopram, EMD 68843, cyanodothepine, citalopram, venlafaxine, milnacipran, duloxetine, desvenlafaxine, cericlamine and ademethionine (preferably s-adenosylmethionine), or a pharmaceutically acceptable salt thereof, and (23) preferably, wherein said SNDRI compound is selected from the group consisting of NS 2330, NS2359, McN 5652, DOV 216,303 and DOV 21,947, or a pharmaceutically acceptable salt thereof.
(24) Finally, the present invention further relates to tablets comprising comprising (a) more than 15 and less than about 20 mg pipamperone, (b) an SNDRI, SNRI or SSRI, (c) and optionally lactose, corn starch, saccharose, talc, and/or magnesium-stearate.

### Detailed description of the invention

Before the present method and products of the invention are described, it is to be understood that this invention is not limited to particular methods, components, products or combinations described, as such methods, components, products and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

All documents cited in the present specification are hereby incorporated by reference in their entirety.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

Depression and anxiety disorders are commonly treated with serotonin re-uptake inhibitors. Unfortunately, however, these compounds can give rise to side effects in use, including sedation. Moreover, a substantial problem in most treatment of mental disorders is the non-response to serotonin re-uptake inhibitors. Also the onset of the therapeutic effect can be delayed undesirable.

A problem to be solved by the present invention is thus the provision of a more efficient therapy and efficient, highly selective and efficacious medicaments for treating mental disorders, in particular depression and anxiety.

Pipamperone has both a selective affinity for the 5-HT2A receptor with a pKi value equal to or higher than 8 towards the 5-HT2A receptor and less than 8 towards other 5HT receptors, and a selective affinity for the D4 receptor with a pKi value equal to or higher than 8 towards the D4 receptor and less than 8 towards other dopamine receptors (see Table 1). A dose response relationship is expected for pipamperone. Efficacy, i.e. antagonistic activity, is expected by at least 20 % receptor occupancy (RO) of the 5-HT_{2A} and D4 receptors. Indeed, according to pKi modelling it was expected that that the optimal dose of pipamperone would be about 7.5 mg per day, while beneficial results would still be achieved from the lower limit of 5 mg per day up to the upper limit of 15 mg per day.

However, adverse effects are expected with more than 10% RO of the α-1 receptor, D2 receptor, or the H1 receptor. Hence, when establishing dissociation constants (*K*_{d}) and pKi modelling, adverse effects are expected at doses higher than 15 mg pipamperone per day.

The present inventor surprisingly found that a simultaneous or foregoing treatment with pipamperone having a high selective 5-HT2A antagonist activity leads to a greater response towards serotonin re-uptake inhibitors, including SSRIs, SNDRIs and SNRIs. However, not all compounds exhibiting 5-HT2A antagonism are useful: competition between 5-HT2A stimulation via serotonin and 5-HT2A antagonism via the compound could be responsible for the lack of more efficacy of compounds which have both a selective serotonin re-uptake inhibitory and 5-HT2A antagonist profile, such as trazodone and nefazodone. In particular, the present inventor surprisingly found that pipamperone at a dose of more than 15 to 20 mg/day in combination with serotonin re-uptake inhibitors, including SSRIs, SNDRIs and SNRIs, has an improved effect in treating mood and anxiety disorders, by an increased D4 and 5-HT_{2A} RO, while adverse effects are absent.

In addition, the present inventor surprisingly found that pipamperone at an unconventionally low dose of about 15 to about 20 mg/day in combination with an SRI is efficacious in treating patients suffering from mood and anxiety disorders, which are treatment refractory to antidepressants and anxiolytic agents, respectively.

The combination treatment and medicaments of the invention provides faster symptom resolution, less residual symptoms and more remission relative to a mono therapy with these compounds.

The term "serotonin re-uptake inhibitor" or "SRI" as used herein refers to any compound elevating the extracellular plasma concentration of serotonin in the synaptic cleft by blocking or inhibiting the function of SERT. The term "SRI" does not exclude the effects of these compounds on any other compound, e.g. other neurotransmitters. In particular, the term "SRI" includes SSRIs, SNDRIs and SNRIs. The serotonin transporter (SERT) is an integral membrane protein that transports the neurotransmitter serotonin from synaptic spaces into presynaptic neurons. This transport of serotonin by the SERT protein terminates the action of serotonin and recycles it in a sodium-dependent manner. SERT belongs to NE, DA, SERT monoamine transporter family, and spans the plasma membrane 12 times.

Mood and anxiety disorders can be diagnosed using criteria found in the American Psychiatric Association's revised fourth edition of the Diagnostic and Statistical Manual of Mental Disorders (DSM-IV-TR), and the WHO's International Statistical Classification of Diseases and Related Health Problems (ICD-10). DSM-IV sets forth diagnostic criteria, descriptions and other information to guide the classification and diagnosis of mental disorders and is commonly used in the field of neuropsychiatry. It is for instance available on the internet under: http://www.behavenet.com/ capsules/disorders/ dsm4tr.htm.

The mood disorders grouped under the DSM-IV include major depressive disorder, dysthymic disorder, bipolar disorder, cyclothymic disorder, mood disorder due to a general medical condition and substance induced mood disorder. For each of these mood disorders there are specific criteria that a person's symptoms must meet in order to receive a diagnosis.

The anxiety disorders grouped under the DSM-IV include panic disorder, phobias, obsessive-compulsive disorder (OCD), posttraumatic stress disorder (PTSD) and generalized anxiety disorder (GAD). For each of these anxiety disorders there are specific criteria that a person's symptoms must meet in order to receive a diagnosis.

The term "depression" according to the invention includes Major depressive disorder, Major depressive episode, Atypical depression, Melancholic depression, Psychotic depression, Depressive Disorder Not Otherwise Specified, Depression (mood), Postpartum depression, Dysthymia, Adjustment disorder with depressed mood, Seasonal affective disorder (SAD), all as known in the art.

Depression can be scored by e.g. the Hamilton Depression Rating Scale (HDRS or HAMD) (Hamilton M. A rating scale for depression. J Neurol Neurosurg Psychiatry 196023:56-62) or the Montgomery-Asberg Depression Rating Scale (abbreviated MADRS). There is, however, a high degree of statistical correlation between scores on the two measures. The Clinical Global Impression (CGI) rating scales are commonly used measures of symptom severity, treatment response and the efficacy of treatments in treatment studies of patients with mental disorders. The CGI - Severity scale (CGI-S) is a 7-point scale which can be used by the clinician to rate the severity of the patient's illness at the time of assessment, relative to the clinician's past experience with patients who have the same diagnosis.

The high selective affinity of pipamperone towards the 5-HT2A receptor and the D4 receptor is reflected in the low dosage which is needed for the treatment of mood or anxiety disorders and also contributes to the efficacy of the treatment with SRIs.

Pipamperone is the conventional name given for the compound of the formula 1'-[3-(p-Fluorobenzoyl)propyl]-[1,4'-bipiperidine]-4'-carboxamide. Pipamperone is also the active ingredient of for instance the commercially available Dipiperon (Janssen, Cilag B.V).

According to the leaflet of the manufacturer, the therapeutic indications for pipamperone are psychoses and the symptomatic treatment of serious forms of agitation and anxiety only. Pipamperone is contraindicated for depression. For adults it is recommended to start with 40 to 80 mg a day. If necessary the dose may be increased to a maximum of 360 mg per day. In conventional pipamperone treatment, the active ingredient is available in tablets of 40 mg per tablet or in solutions of 2 mg per drop. Conventional usage of high doses ranging from 40 to 360 mg is prescribed. For instance, for children up to the age of 14, doses corresponding with 2 to 6 mg per kg body weight are conventionally prescribed.

Preferred dosages which, according to the invention, have been shown to be effective for treating the mood or anxiety disorder in combination with serotonin re-uptake inhibitors, including SSRIs, SNDRIs and SNRIs, for instance, (es)citalopram, range between more than 15 up to 20, but preferably less than 20 mg pipamperone per day. More preferably, dosages of 15, 15.1, 15.2, 15.3, 15.4, 15.5, 16, 16.5, 17, 17.5, 18, 18.5, 19, 19.5, 19.9 and 20 mg pipamperone per day are used in the treatment of mood and anxiety disorders, such as depression.

In a preferred embodiment, dosages of more than 15 mg to about 17.5 mg per day are used in the treatment of mood and anxiety disorders, preferably in treating children and elderly patients, in particular, about 15, 15.1, 15.2, 15.3, 15.4, 15.5, 16, 16.5, 17, 17.5 mg pipamperone per day is used in the treatment of anxiety and mood disorders, such as depression.

In a further preferred embodiment, dosages of about 17.5 mg to about 20 mg per day are used in the treatment of anxiety and mood disorders, such as depression, preferably in treating adults. Preferably, about 17.5, 18, 18.5, 19, 19.5, 19.9 and 20 mg pipamperone per day is used in the treatment of anxiety and mood disorders, such as depression.

Dosages between about 0.175 mg and about 0.25 mg pipamperone per kg bodyweight per day are used in the treatment of anxiety and mood disorders, such as depression, such as, for instance, 0.175, 0.18, 0.185, 0.19, 0.195, 0.20, 0.21, 0.22, 0.225, 0.23, 0.24, 0.245 and 0.25 mg pipamperone per day per kg bodyweight is used.

It will be appreciated that the daily doses may be unitary doses.

Citalopram or citalopram hydrobromide is a selective serotonin (5-hydroxytryptamine / 5-HT) re-uptake inhibitor (SSRI) and is the conventional name given for the compound of the formula (RS)-1-[3-(dimethylamino)propyl]-1-(p-flurophenyl)-5-phthalancarbonitrile hydro-bromide. Citalopram is an antidepressant drug used to treat depression associated with mood disorders. Citalopram is also known as Celexa, Cipramil, Citrol, Seropram, Talam, Citabax, Citaxin, Citalec, Recital, Zetalo, Celapram, Ciazil, Zentius, Ciprapine, Cilift, Citox and Cipram. Escitalopram (trade names Lexapro, Cipralex, Sipralexa) is the pure (S) enantiomer of racemic citalopram. The recommended starting dose is 10 mg per day which gradually increased. This implies that adjustment of the dose is not necessary, that the effect cannot be augmented and that the onset of the effect cannot be reduced.

Nevertheless, the present inventor found that the combination with the low dose of pipamperone of the present invention results in an earlier onset of the effect, the effect was ameliorated, and at least the same effect was obtained with a lower dose of (es)citalopram, resulting in less side-effects. It will be understood that the term (es)citalopram relates to escitalopram and/or citalopram.

According to an embodiment, a daily dose of the active ingredient of (es)citalopram, ranges between 5 and 60 mg per day. Preferably, daily doses of active ingredient ranging between 20 and 40 mg per day are administered. More preferably, a daily dose of 5, 7.5, 10, 15, 20, 25, 30, 35, 40, 45, 50, 55 or 60 mg per day is administered. In view of the augmenting effect, including providing a faster onset, of pipamperone, the preferred daily dose of the active ingredient may range between 5 and 30 mg per day.

The present inventors found that pipamperone which binds to the 5-HT2A receptor with a pKi of at least 8 but for which the binding affinity, i.e. pKi, towards other 5HT receptors is less than 8 in combination with a high selective affinity for the D4 receptor, i.e. which bind to the D4 receptor with a pKi of at least 8 but for which the binding affinity, i.e. pKi, towards other dopamine receptors is less than 8 also show such an improved effect on serotonin re-uptake inhibitors, including SSRIs, SNDRIs and SNRIs, and in particular (es)citalopram in the treatment of mood disorders and anxiety disorders, and in particular depression.

The term "other 5HT receptors" as used herein relate to, for instance, 5-HT₁ receptors (e.g. 5-HT_{1A}, 5-HT_{1B}, 5-HT_{1D}, 5-HT_{1E}, 5-HT_{1F}), 5-HT_{2B}, 5-HT_{2C}, 5-HT₆ (rat) and 5-HT₇ (rat). The expression "selective affinity for the 5-HT_{2A} receptor" relates to a receptor having a higher affinity for the 5-HT_{2A} receptor than for other 5-HT receptors. The expression "selective affinity for the D4 receptor" means that the receptor has a higher affinity for the dopamine D4 receptor than for other dopamine receptors. The term "other dopamine receptors" relates to, for instance, D1, D2 and D3 dopamine receptors. pKi values of test compounds for dopamine receptors as well as 5-HT2A receptors can be measured using commonly known assays.

Table 1 illustrates the selective affinity of for instance pipamperone for the 5-HT_{2A} and for the D4 receptor. In addition, Table 1 also illustrates the low or absence of affinity of pipamperone for other receptors such as the adrenergic receptors α-1A, α-2A, α-2B, α-2C, β-1, β-2, and the histamine receptor H1. As such, treating patients with pipamperone will provide for less side-effects which otherwise result from simultaneous stimulation of other receptors.

The low dosage which can be used in pipamperone treatment, as already described earlier, contributes to the high selective affinity of the compound towards the 5-HT_{2A} receptor and the D4 receptor and therefore also to the efficacy of the treatment.

As such, in a combination treatment, the doses of the compounds listed in Table 3 for treating patients with mental disorders may be decreased to about 10 - 90% of the conventional dose, preferably to about 20 - 80%, or 30 - 70%, or 40 - 60% or to about 50% of the conventional dose. Even if the administered dose of the serotonin re-uptake inhibitors, including SSRIs, SNDRIs and SNRIs, and in particular (es)citalopram is decreased in the combination therapy, the therapeutic effect may be sustained or ameliorated relative to the conventional dose. The danger of side effects of a treatment with serotonin re-uptake inhibitors, including SSRIs, SNDRIs and SNRIs, and in particular (es)citalopram, can be decreased or minimized in the combination therapy of the invention. In this regard, the term conventional dose refers to the dose used contemporaneously for serotonin re-uptake inhibitors, including SSRIs, SNDRIs and SNRIs, and in particular (es)citalopram, in the treatment of mood disorders and anxiety disorders, for instance, according to the supplier's or physician's description.

The terms "treatment", "treating", and the like, as used herein include amelioration or elimination of a developed mental disease or condition, in particular depression, once it has been established or alleviation of the characteristic symptoms of such disease or condition. As used herein these terms also encompass, depending on the condition of the patient, preventing the onset of depression or of symptoms associated with depression, including reducing the severity of depression or symptoms associated therewith prior to affliction with said depression. Such prevention or reduction prior to affliction refers to administration of the compound or composition of the invention to a patient that is not at the time of administration afflicted with depression. "Preventing" also encompasses preventing the recurrence or relapse-prevention of depression or of symptoms associated therewith, for instance after a period of improvement. It should be clear that mental conditions, e.g. depression, may be responsible for physical complaints. In this respect, the term "treating" also includes prevention of a physical disease or condition or amelioration or elimination of the developed physical disease or condition once it has been established or alleviation of the characteristic symptoms of such conditions.

As used herein, the term "medicament" also encompasses the terms "drug", "therapeutic", "potion" or other terms which are used in the field of medicine to indicate a preparation with therapeutic or prophylactic effect.

According to a further embodiment, the invention relates to the use of pipamperone for the preparation of a medicament for treating mood disorders or anxiety disorders, and in particular depression whereby pipamperone is administered simultaneously with, separate from or sequential to said serotonin re-uptake inhibitors, including SSRIs, SNDRIs and SNRIs, and in particular (es)citalopram, to augment the therapeutic effect of said serotonin re-uptake inhibitors, including SSRIs, SNDRIs and SNRIs, and in particular (es)citalopram in the treatment of mood disorders and anxiety disorders, and in particular depression, or to provide a faster onset of the therapeutic effect of said serotonin re-uptake inhibitors, including SSRIs, SNDRIs and SNRIs, and in particular (es)citalopram in the treatment of said mood disorders and anxiety disorders, and in particular depression.

When pipamperone is administered prior to serotonin re-uptake inhibitors, including SSRIs, SNDRIs and SNRIs, and in particular (es)citalopram, pipamperone is administered at least during 1 day prior to said second compound, i.e. serotonin re-uptake inhibitors, including SSRIs, SNDRIs and SNRIs, and in particular (es)citalopram. Preferably, pipamperone is administered for at least 1, 2, 3, 4, 5, 6, 7, 8, 9, 10, 11, 12, 13 or 14 days prior to the administration of serotonin re-uptake inhibitors, including SSRIs, SNDRIs and SNRIs, and in particular (es)citalopram. Preferably, pipamperone is administered for at least 2, 3, 4 or 5 weeks prior to the administration of serotonin re-uptake inhibitors, including SSRIs, SNDRIs and SNRIs, and in particular (es)citalopram.

Preferably, pipamperone and serotonin re-uptake inhibitors, including SSRIs, SNDRIs and SNRIs, and in particular (es)citalopram are administered within 24 hours, e.g. on the same day, even more preferably within 16 hours, or even more preferably within 14, 12, 10, 9, 8, 7, 6, 5, 4, 3, 2 or 1 hour, or even less.

Pipamperone is preferably administered once a day. In a further embodiment, pipamperone is administered 2, or 3 or even 4 times a day. It will be understood that, apart from daily doses, the compounds can be administered by other schedules. For instance, the present invention also contemplates depot injection, in which a long acting form of the active compound is injected into the body, such as the muscles. From there the active compound slowly enters the rest of the body, so one injection can last from 1 to 4 weeks or even multiple months. Other form of dosage administrations relate to "once-α-week" pills, in which the ingredient is slowly released over a period of a week, and slow-release patches, e.g. a CDS (Continuous Delivery System), or Once-a-Day Transdermal Patches.

It will be understood that the total dose of pipamperone per day of any dosage regime is as described above, e.g. from about 15 mg per day to about 20 mg pipamperone per day. In the present invention, pipamperone and serotonin re-uptake inhibitors, including SSRIs, SNDRIs and SNRIs, and in particular (es)citalopram, may be present a single composition or in separate compositions. The compositions according to the invention may be pharmaceutical compositions or formulations.

When pipamperone is administered simultaneously with, separate from or sequential to , particularly separate from or sequential to said serotonin re-uptake inhibitors, including SSRIs, SNDRIs and SNRIs, and in particular (es)citalopram, said pipamperone and said serotonin re-uptake inhibitors, including SSRIs, SNDRIs and SNRIs, and in particular (es)citalopram, e.g. the active ingredients thereof, are administered in separate pharmaceutical compositions or formulations. Said separate pharmaceutical compositions or formulations of either pipamperone or serotonin re-uptake inhibitors, including SSRIs, SNDRIs and SNRIs, and in particular (es)citalopram can be individually packed. Preferably, said separate pharmaceutical compositions or formulations are packed together, *i*.*e*. combined preparations, such as for instance in one box, on one strip, e.g. blister strip, etc. Said separate pharmaceutical compositions or formulations are preferred in adjusting individual doses of either or both compounds, within the ranges of the invention. Accordingly, the invention relates to combined preparations comprising a pharmaceutical composition comprising pipamperone, and a pharmaceutical composition comprising an SRI as defined herein, for simultaneous, separate or sequential use for treating a mood disorder or anxiety disorder, wherein said pipamperone is to be administered to a patient in a daily dose ranging between more than 15 and about 20 mg of the active ingredient.

When pipamperone is administered simultaneously with said serotonin re-uptake inhibitors, including SSRIs, SNDRIs and SNRIs, and in particular (es)citalopram, said pipamperone and said serotonin re-uptake inhibitors, including SSRIs, SNDRIs and SNRIs, and in particular (es)citalopram, e.g. the active ingredients thereof, are administered in separate pharmaceutical compositions or formulations, or in pharmaceutical compositions or formulations comprising both elements, i.e. said pipamperone and said serotonin re-uptake inhibitors, including SSRIs, SNDRIs and SNRIs, and in particular (es)citalopram, such as, for instance, combination pharmaceutical compositions or combination formulations. Preferably, said combination is a pill, powder or solution comprising both pipamperone and said serotonin re-uptake inhibitors, including SSRIs, SNDRIs and SNRIs, and in particular (es)citalopram, e.g. at the doses of the invention, for ease in administration and management.

It has been found that the low dose pipamperone augments the therapeutic effect of and/or provides a faster onset of serotonin re-uptake inhibitors, including SSRIs, SNDRIs and SNRIs, and in particular (es)citalopram in mood disorders, such as depression, or anxiety disorders. It is further demonstrated that the combination treatment of the present invention provides at least a similar therapeutic effect with lower doses of serotonin re-uptake inhibitors, including SSRIs, SNDRIs and SNRIs, and in particular (es)citalopram in mood and anxiety disorders, such as depression, relative to the conventional doses of serotonin re-uptake inhibitors, including SSRIs, SNDRIs and SNRIs, and in particular (es)citalopram, decreasing side effects. Moreover, this combination treatment is especially beneficiary in elderly and children.

The amount of pipamperone required to produce the efficacious effect will, of course, vary and is ultimately at the discretion of the medical practitioner. The factors to be considered include the route of administration and nature of the formulation, the patient's body weight, age and general condition and the nature and severity of the disease to be treated.

More commonly these days, pharmaceutical formulations are prescribed to the patient in "patient packs" containing the whole course of treatment in a single package, usually a blister pack. Patient packs have an advantage over traditional prescriptions, where a pharmacist divides a patient's supply of a pharmaceutical from a bulk supply, in that the patient always has access to the package insert contained in the patient pack, normally missing in traditional prescriptions. The inclusion of a package insert has been shown to improve the patient's compliance with the physician's instructions.

It will be understood that the administration of the combination of the invention by means of a single patient pack, or patient packs of each formulation, with a package insert directing the patient to the correct use of the invention is a desirable additional feature of this invention.

Suitable dosage units of pipamperone are for instance, more than 15 to less than 20 mg and suitable dosage units containing an SRI are, for instance, 0.1 to 300 mg.

According to a further aspect of the invention, there is provided a patient pack comprising at least one active ingredient of the combination of the invention and an information insert containing directions on the use of the combination of the invention.

According to another aspect the invention provides a double pack comprising in association for separate administration of either pipamperone or an SRI.

According to a further aspect, the present invention relates to pharmaceutical compositions or formulations comprising both pipamperone at a dose of between more than about 15 mg and less than about 17.5 mg, more preferably, dosages of 15, 15.1, 15.2, 15.3, 15.4, 15.5, 16, 16.5, 17, and 17.5 mg and an SRI, such as SSRI, SNRI or SNDRI, as well as the use thereof per day, preferably in treating children and elderly patients. Preferably, said SSRI is (es)citalopram at a range of between 5 and 60 mg, preferably, the active ingredient ranging between 20 and 35 mg, even more preferably about 5, 7.5, 10, 15, 20, 25, 30, 30, 40, 45, 50, 55 or 60 mg.

Accordingly, the present invention relates to pharmaceutical compositions or formulations comprising both pipamperone at a dose of between more than about 17.5 mg and less than about 20 mg, more preferably, dosages of 17.5, 18, 18.5, 19, 19.5, 19.9 and 20 mg and an SRI, such as SSRI, SNRI or SNDRI, as well as the use thereof per day, preferably in treating adults. Preferably, said SSRI is (es)citalopram at a range of between 5 and 60 mg, preferably, the active ingredient ranging between 20 and 35 mg, even more preferably about 5, 7.5, 10, 15, 20, 25, 30, 35, 40, 45, 50, 55 or 60 mg.

The compounds according to the invention may be chemical or biological in nature, or may be chemically synthesised.

In this invention, the term "antagonist" refers to an interaction between chemicals in which one partially or completely inhibits the effect of the other, in particular agents having high affinity for a given receptor, but which do not activate this receptor. In this invention, the term "inverse agonist" refers to a ligand which produces an effect opposite to that of the agonist by occupying the same receptor. In this invention, the term "agonist" relates to an agent which both binds to a receptor and has an intrinsic effect. In this invention, the term "partial agonist" relates to an agent with lower intrinsic activity than a full agonist, and which produces a lower maximum effect.

The term "active metabolite" as used herein relates to a therapeutically active compound produced by the metabolism of a parent drug. Drugs administered to treat diseases are usually transformed (metabolized) within the body into a variety of related chemical forms (metabolites), some of which may have therapeutic activity (an active metabolite).

The present invention also encompasses the use of pipamperone and/or serotonin re-uptake inhibitors, including SSRIs, SNDRIs and SNRIs, and in particular (es)citalopram, administered in the form of a pharmaceutically acceptable salt in admixture with a suitable pharmaceutically acceptable excipient.

To prepare the pharmaceutical compositions, comprising either or both of pipamperone and/or serotonin re-uptake inhibitors, including SSRIs, SNDRIs and SNRIs, and in particular (es)citalopram, *i*.*e*. combination pharmaceutical composition, an effective amount of the active ingredients, in acid or base addition salt form or base form, is combined in admixture with a pharmaceutically acceptable carrier, which can take a wide variety of forms depending on the form of preparation desired for administration. These pharmaceutical compositions are desirably in unitary dosage form suitable, for administration orally, nasal, rectally, percutaneously, transdermally, by parenteral, intramuscular, intravascular injection or intrathecal administration. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included.

The pharmaceutical compounds for treatment are intended for parenteral, topical, oral or local administration and generally comprise a pharmaceutically acceptable carrier and an amount of the active ingredient sufficient to reverse or prevent the bad effects of mental disorders. The carrier may be any of those conventionally used and is limited only by chemico-physical considerations, such as solubility and lack of reactivity with the compound, and by the route of administration.

Preferably, the compounds of the invention are provided as a pharmaceutically acceptable salt. Examples of pharmaceutically acceptable acid addition salts for use in the present inventive pharmaceutical composition include those derived from mineral acids, such as hydrochloric, hydrobromic, phosphoric, metaphosphoric, nitric and sulfuric acids, and organic acids, such as tartaric, acetic, citric, malic, lactic, fumaric, benzoic, glycolic, gluconic, succinic, p-toluenesulphonic acids, and arylsulphonic, for example. The present invention relates specifically to pipamperone and the use thereof, **characterised in that** said pipamperone is pipamperone dihydrochloride or pipamperone acetate.

The pharmaceutically acceptable excipients described herein, for example, vehicles, adjuvants, carriers or diluents, are well-known to those who are skilled in the art and are readily available to the public. It is preferred that the pharmaceutically acceptable carrier be one that is chemically inert to the active compounds and one that has no detrimental side effects or toxicity under the conditions of use.

The following formulations for oral, aerosol, parenteral, subcutaneous, intravenous, intramuscular, interperitoneal, rectal, and vaginal administration are merely exemplary and are in no way limiting. Overall, the requirements for effective pharmaceutical carriers for parenteral compositions are well known to those of ordinary skill in the art. See Pharmaceutics and Pharmacy Practice, J.B. Lippincott Company, Philadelphia, PA, Banker and Chalmers, eds., pages 238-250, (1982), and ASHP Handbook on Injectable Drugs, Toissel, 4th ed., pages 622-630 (1986). Topical formulations, including those that are useful for transdermal drug release, are well-known to those of skill in the art and are suitable in the context of the present invention for application to skin.

Formulations, comprising either or both of pipamperone and/or serotonin re-uptake inhibitors, including SSRIs, SNDRIs and SNRIs, and in particular (es)citalopram, *i.e.* combination formulations, suitable for oral administration require extra considerations considering the nature of the compounds and the possible breakdown thereof if such compounds are administered orally without protecting them from the digestive secretions of the gastrointestinal tract. Such a formulation can consist of (a) liquid solutions, such as an effective amount of the compound dissolved in diluents, such as water, saline, or orange juice; (b) capsules, sachets, tablets, lozenges, and troches, each containing a predetermined amount of the active ingredient, as solids or granules; (c) powders; (d) suspensions in an appropriate liquid; and (e) suitable emulsions. Liquid formulations may include diluents, such as water and alcohols, for example, ethanol, benzyl alcohol, and the polyethylene alcohols, either with or without the addition of a pharmaceutically acceptable surfactant, suspending agent, or emulsifying agent. Capsule forms can be of the ordinary hard- or soft-shelled gelatin type containing, for example, surfactants, lubricants, and inert fillers, such as lactose, sucrose, calcium phosphate, and corn starch. Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

The compounds of the present invention, *i.e.* pipamperone and/or serotonin re-uptake inhibitors, including SSRIs, SNDRIs and SNRIs, and in particular (es)citalopram alone or in combination with other suitable components, can be made into aerosol formulations to be administered via inhalation. For aerosol administration, the compounds are preferably supplied in finely divided form along with a surfactant and propellant. Typical percentages of compounds are 0.01%-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the compounds, preferably 0.25-5%. The balance of the compounds is ordinarily propellant. A carrier can also be included as desired, e.g., lecithin for intranasal delivery. These aerosol formulations can be placed into acceptable pressurized propellants, such as dichlorodifluoromethane, propane, nitrogen, and the like. They also may be formulated as pharmaceuticals for non-pressured preparations, such as in a nebulizer or an atomizer. Such spray formulations may be used to spray mucosa.

It should be clear that the compounds and compositions described herein are useful for treating any patient in need thereof, in particular humans.

Patients receiving antidepressant monotherapy may be or become partially or totally resistant to treatment in 10 to 30 percent of cases. "Pooping-out" can occur with any conventional antidepressant. Relapse or recurrence of depression while still taking medication (*i*.*e*., breakthrough) can also occur. While there is no definitive answer as to why this happens, it may be a case of the patient developing tolerance to the drug. The most commonly strategies used to deal with this problem include augmentation with a second drug, raising the dose or switching to another drug entirely.

A change from one antidepressant to another in the same or different class has not produced impressive response rates among depressed patients. If drug-switching is chosen as the method of therapy, patients should be closely monitored for possible drug interactions or other adverse effects. This is particularly true if the half-life of the first agent is quite long (e.g., fluoxetine [Prozac]) and another SSRI is begun before a prudent "washout" period has occurred. This situation may sometimes result in serotonin syndrome (toxic levels of central nervous system serotonin that result in hyperalertness, agitation, confusion, restlessness, myoclonus, hyperreflexia, diaphoresis, shivering, tremor and, possibly, death.)

The present inventors found that pipamperone which binds to the 5-HT2A receptor with a pKi of at least 8 but for which the binding affinity, i.e. pKi, towards other 5HT receptors is less than 8 in combination with a high selective affinity for the D4 receptor, i.e. which bind to the D4 receptor with a pKi of at least 8 but for which the binding affinity, i.e. pKi, towards other dopamine receptors is less than 8 also show an improved effect on treatment refractory patients with mood disorders or anxiety disorders. Because of high specific binding affinity, pipamperone can be used at low doses. At these low doses, adverse effects are minimized and/or precluded (see e.g. W02005/053796, which is specifically incorporated by reference in its entirety).

Accordingly, the present invention relates to the use as described herein, **characterised in that** the pharmaceutical composition is for treatment of patients who have failed to respond to initial treatment with an antidepressant and/or anxiolytic agent, including an SSRI, SNDRI or SNRI. More in particular, the present invention relates to the use as described above, **characterised in that** the pharmaceutical composition is for treatment of patients with major depression disorder who have failed to respond to initial treatment with an antidepressant or anxiolytic agent, including an SSRI, SNDRI or SNRI. Accordingly, the present invention relates to the use as described herein, **characterised in that** the pharmaceutical composition is for treatment of treatment refractory patients to an antidepressant or anxiolytic agent, including an SSRI, SNDRI or SNRI. More in particular, the present invention relates to the use as described above, **characterised in that** the pharmaceutical composition is for treatment of major depression in treatment refractory patients to an antidepressant or anxiolytic agent, including an SSRI, SNDRI or SNRI.

The term "treatment refractory" patient is used as common in the art, such as, for instance, as understood by the clinician or physician monitoring and/or treating a patient. This term includes patients who have failed to respond to initial treatment, patients who are partially or totally resistant to treatment, patients with recurrent mood or anxiety disorder, and patients pooping-out. Patients who have failed to respond to initial treatment, include patients not ameliorating after for instance 2 weeks of treatment, as diagnosed by criteria described above.

The present invention is now described in more detail by the following embodiments.

Mood and anxiety disorders, in particular depression, can be treated using compounds having a high selective affinity for the 5-HT2A and D4 receptor, for instance pipamperone, in a combination therapy with serotonin re-uptake inhibitors, including SSRIs, SNDRIs and SNRIs, and in particular (es)citalopram.

The present invention thus relates to the use of pipamperone or a pharmaceutically acceptable salt thereof for the preparation of a medicament for treating mood or anxiety disorders, including depression, characterized in that pipamperone or said pharmaceutically acceptable salt thereof is administered simultaneously with, separate from or prior to the administration of serotonin re-uptake inhibitors, including SSRIs, SNDRIs and SNRIs, and in particular (es)citalopram to augment the therapeutic effect or to provide a faster onset of the therapeutic effect of said serotonin re-uptake inhibitors, including SSRIs, SNDRIs and SNRIs, and in particular (es)citalopram, further characterized in that pipamperone is to be administered to a patient in a daily dose ranging between more than 15 and about 20 mg of the active ingredient.

The present invention thus relates to the use of pipamperone or a pharmaceutically acceptable salt thereof for the preparation of a medicament for treating mood or anxiety disorders, including depression, characterized in that pipamperone or said pharmaceutically acceptable salt thereof is administered simultaneously with, separate from or prior to the administration of (es)citalopram to augment the therapeutic effect or to provide a faster onset of the therapeutic effect of said (es)citalopram, further characterized in that pipamperone is to be administered to a patient in a daily dose ranging between more than 15 and about 20 mg of the active ingredient.

According to a preferred embodiment, the invention relates to the uses as described above, wherein said citalopram is escitalopram, or a pro-drug or an active metabolite thereof, or a pharmaceutically acceptable salt thereof. More preferably, said escitalopram and is to be administered in a daily dose ranging between 5 and 50 mg of the active ingredient.

The invention also relates to a pharmaceutical composition or formulation comprising (a) pipamperone, and (b) (es)citalopram, or a pro-drug or an active metabolite thereof, or a pharmaceutically acceptable salt thereof, as a combined preparation for simultaneous, separate or sequential use for treating mood or anxiety disorders, including depression. The invention also relates to a pharmaceutical composition or formulation as described above wherein pipamperone is provided in a unitary dose of between more than 15 and about 20 mg of the active ingredient and wherein said escitalopram, preferably provided in a unitary dose of between 5 and 50 mg of the active ingredient.

The invention also relates to a pharmaceutical composition or formulation as described above wherein pipamperone is provided in a unitary dose of between more than 15 and about 20 mg of the active ingredient and wherein said citalopram is provided in a unitary dose of between 5 and 60 mg of the active ingredient.

The disclosure of all patents, publications (including published patent publications), and database accession numbers and depository accession numbers referenced in this specification are specifically incorporated herein by reference in their entirety to the same extent as if each such individual patent, publication, and database accession number, and depository accession number were specifically and individually indicated to be incorporated by reference.

The mental disorders which can be treated using pipamperone in a combination therapy with an SSRI compound are chosen from mood disorders and anxiety disorders.

The present invention thus relates to the use of pipamperone or a pharmaceutically acceptable salt thereof for the preparation of a medicament for treating mood disorders or anxiety disorders, characterized in that pipamperone or said pharmaceutically acceptable salt thereof is administered simultaneously with, separate from or prior to the administration of a selective serotonin re-uptake inhibitor (SSRI) compound to augment the therapeutic effect or to provide a faster onset of the therapeutic effect of said selective serotonin re-uptake inhibitor (SSRI) compound, further characterized in that pipamperone is to be administered to a patient in a daily dose ranging between 15, preferably more than 15, and about 20 mg of the active ingredient.

According to a preferred embodiment, the invention relates to the uses as described above, wherein said SSRI compound is selected from the group consisting of YM 992, VPI-013 (also known as OPC-14523), sertraline, paroxetine, LY 214.281, LU AA 21-004, Lu 35-138, litoxetine, ifoxetine, fluvoxamine (controlled release formulation), fluvoxamine, fluoxetine, femoxetine, escitalopram, EMD 68843, cyanodothepine, citalopram, cericlamine and ademethionine (preferably s-adenosylmethionine), milnacipran venlafaxine, duloxetine, desvenlafaxine or a pro-drug or an active metabolite thereof, or a pharmaceutically acceptable salt thereof. Even more preferably, said SSRI compound is chosen from the group consisting of litoxetine, fluvoxamine (controlled release formulation), citalopram and escitalopram, or a pro-drug or an active metabolite thereof, or a pharmaceutically acceptable salt thereof.

More preferably, said SSRI compound is a compound detailed in Table 3 and is to be administered in a daily dose ranging between the amounts of the active ingredient also as detailed in Table 3; preferably, said SSRI compound is paroxetine and is to be administered in a daily dose ranging between 20 and 75 mg of the active ingredient, said SSRI compound is escitalopram and is to be administered in a daily dose ranging between 5 and 50 mg of the active ingredient, said SSRI compound is citalopram and is to be administered in a daily dose ranging between 5 and 60 mg of the active ingredient, said SSRI compound is fluoxetine and is to be administered in a daily dose ranging between 20 and 60 mg of the active ingredient, said SSRI compound is fluvoxamine and is to be administered in a daily dose ranging between 50 and 300 mg of the active ingredient, said SSRI compound is sertraline and is to be administered in a daily dose ranging between 25 and 300 mg of the active ingredient, said SSRI compound is fluvoxamine (controlled release formulation) and is to be administered in a daily dose ranging between 100 and 300 mg of the active ingredient, said SSRI compound is milnacipran and is to be administered in a daily dose ranging between 50 and 200 mg of the active ingredient, said SSRI compound is venlafaxine and is to be administered in a daily dose ranging between 75 and 300 mg of the active ingredient, said SSRI compound is duloxetine and is to be administered in a daily dose ranging between 40 and 120 mg of the active ingredient, and/or said SSRI compound is desvenlafaxine and is to be administered in a daily dose ranging between 50 and 250 mg of the active ingredient.

The invention also relates to a pharmaceutical composition comprising (a) pipamperone in a dose ranging between 15, preferably more than 15, and about 20 mg of the active ingredient, and (b) an SSRI compound, preferably selected from the group consisting of YM 992, VPI-013 (also known as OPC-14523), sertraline, paroxetine, LY 214.281, LU AA 21-004, Lu 35-138, litoxetine, ifoxetine, fluvoxamine (controlled release formulation), fluvoxamine, fluoxetine, femoxetine, escitalopram, EMD 68843, cyanodothepine, citalopram, venlafaxine, milnacipran, duloxetine, desvenlafaxine, cericlamine and ademethionine (preferably s-adenosyl-methionine), or a pro-drug or an active metabolite thereof, or a pharmaceutically acceptable salt thereof, as a combined preparation for simultaneous, separate or sequential use for treating mood disorders or anxiety disorders. The invention also relates to a pharmaceutical composition comprising (a) pipamperone in a dose ranging between 15, preferably more than 15, and about 20 mg of the active ingredient, and (b) an SSRI compound, preferably selected from the group consisting of YM 992, VPI-013 (also known as OPC-14523), sertraline, paroxetine, LY 214.281, LU AA 21-004, Lu 35-138, litoxetine, ifoxetine, fluvoxamine (controlled release formulation), fluvoxamine, fluoxetine, femoxetine, escitalopram, EMD 68843, cyanodothepine, citalopram, venlafaxine, milnacipran, duloxetine, desvenlafaxine, cericlamine and ademethionine (preferably s-adenosyl-methionine), or a pro-drug or an active metabolite thereof, or a pharmaceutically acceptable salt thereof.

The invention also relates to a pharmaceutical composition as described above wherein pipamperone is provided in a unitary dose ranging between 15, preferably more than 15, and about 20 mg of the active ingredient and wherein said SSRI compound is paroxetine, preferably provided in a unitary dose of between 20 and 75 mg of the active ingredient.

The invention also relates to a pharmaceutical composition as described above wherein pipamperone is provided in a unitary dose ranging between 15, preferably more than 15, and about 20 mg of the active ingredient and wherein said SSRI compound is escitalopram, preferably provided in a unitary dose of between 5 and 50 mg of the active ingredient.

The invention also relates to a pharmaceutical composition as described above wherein pipamperone is provided in a unitary dose ranging between 15, preferably more than 15, and about 20 mg of the active ingredient and wherein said SSRI compound is citalopram, preferably provided in a unitary dose of between 5 and 60 mg of the active ingredient.

The invention also relates to a pharmaceutical composition as described above wherein pipamperone is provided in a unitary dose ranging between 15, preferably more than 15, and about 20 mg of the active ingredient and wherein said SSRI compound is fluoxetine, preferably provided in a unitary dose of between 20 and 60 mg of the active ingredient.

The invention also relates to a pharmaceutical composition as described above wherein pipamperone is provided in a unitary dose ranging between 15, preferably more than 15, and about 20 mg of the active ingredient and wherein said SSRI compound is fluvoxamine, preferably provided in a unitary dose of between 50 and 300 mg of the active ingredient.

The invention also relates to a pharmaceutical composition as described above wherein pipamperone is provided in a unitary dose ranging between 15, preferably more than 15, and about 20 mg of the active ingredient and wherein said SSRI compound is sertraline, preferably provided in a unitary dose of between 25 and 300 mg of the active ingredient.

The invention also relates to a pharmaceutical composition as described above wherein pipamperone is provided in a unitary dose ranging between 15, preferably more than 15, and about 20 mg of the active ingredient and wherein said SSRI compound is fluvoxamine (controlled release formulation), preferably provided in a unitary dose of between 100 and 300 mg of the active ingredient.

The invention also relates to a pharmaceutical composition as described above wherein pipamperone is provided in a unitary dose ranging between 15, preferably more than 15, and about 20 mg of the active ingredient and wherein said SSRI compound is milnacipran, preferably provided in a unitary dose of between 50 and 200 mg of the active ingredient.

The invention also relates to a pharmaceutical composition as described above wherein pipamperone is provided in a unitary dose ranging between 15, preferably more than 15, and about 20 mg of the active ingredient and wherein said SSRI compound is venlafaxine, preferably provided in a unitary dose of between 75 and 300 mg of the active ingredient.

The invention also relates to a pharmaceutical composition as described above wherein pipamperone is provided in a unitary dose ranging between 15, preferably more than 15, and about 20 mg of the active ingredient and wherein said SSRI compound is duloxetine, preferably provided in a unitary dose of between 40 and 120 mg of the active ingredient.

The invention also relates to a pharmaceutical composition as described above wherein pipamperone is provided in a unitary dose ranging between 15, preferably more than 15, and about 20 mg of the active ingredient and wherein said SSRI compound is desvenlafaxine, preferably provided in a unitary dose of between 50 and 250 mg of the active ingredient.

According to an embodiment, a daily doses of active ingredient of SSRI, preferably paroxetine, ranges between 12.5 and 75 mg day. Preferably, daily doses of active ingredient ranging between 25 and 60 mg are administered. More preferably, a daily dose of 12.5, 15, 20, 25, 30, 35, 40, 45, 50, 55, 60, 65, 70 or 75 mg is administered.

According to an embodiment, a daily dose of active ingredient of SSRI, preferably escitalopram, ranges between 5 and 50 mg. Preferably, daily doses of active ingredient ranging between 12.5 and 25 mg are administered. More preferably, a daily dose of 5, 7.5, 10, 12.5, 15, 17.5, 20, 25, 30, 35, 40, 45 or 50 mg is administered.

According to an embodiment, a daily doses of active ingredient of SSRI, preferably citalopram, ranges between 5 and 60 mg. Preferably, daily doses of active ingredient ranging between 20 and 40 mg are administered. More preferably, a daily dose of 5, 7.5, 10, 15, 20, 25, 30, 35, 40, 45, 50, 55 or 60 mg is administered.

According to an embodiment, a daily dose of active ingredient of SSRI, preferably Fluoxetine (Prozac), ranges between 20 and 60 mg. Preferably, daily doses of active ingredient ranging between 30 and 50 mg are administered. More preferably, a daily dose of 20, 25, 30, 35, 40, 45, 50, 55 or 60 mg is administered.

Fluvoxamine or fluvoxamine maleate (luvox, fevarin) is a selective serotonin (5-HT) re-uptake inhibitor (SSRI) belonging to a new chemical series, the 2-aminoethyl oxime ethers of aralkylketones. It is chemically unrelated to other SSRIs and clomipramine. It is chemically designated as 5-methoxy-4'-(trifluoromethyl) valerophenone (E)-O-(2-aminoethyl) oxime maleate (1:1).

According to an embodiment, a daily dose of active ingredient of fluvoxamine ranges between 50 and 300 mg. Preferably, daily doses of active ingredient ranging between 100 and 200 mg are administered. More preferably, a daily dose of 50, 75, 100, 150, 200, 250 or 300 mg is administered.

According to an embodiment, a daily dose of active ingredient of fluvoxamine in a controlled release mode ranges between 100 and 300 mg per day. Preferably, daily doses of active ingredient ranging between 150 and 200 mg per day are administered in a controlled release mode. More preferably, a daily dose of 100, 150, 200, 250 or 300 mg per day is administered by controlled release.

According to an embodiment, a daily dose of active ingredient of sertraline ranges between 25 and 300 mg. Preferably, daily doses of active ingredient ranging between 50 and 150 mg are administered. More preferably, a daily dose of 25, 50, 75, 100, 150, 175, 200, 225, 250, 275 or 300 mg is administered.

According to an embodiment, a daily dose of active ingredient of milnacipran ranges between 50 and 200 mg are administered. More preferably, a daily dose of 50, 75, 100, 125, 150, 175 or 200 mg is administered.

According to an embodiment, a daily dose of active ingredient of venlafaxine ranges between 75 and 300 are administered. More preferably, a daily dose of 75, 100, 125, 150, 175, 200, 225, 250, 275 or 300 mg is administered.

According to an embodiment, a daily dose of active ingredient of duloxetine ranges between 40 and 120 mg are administered. More preferably, a daily dose of 40, 45, 50, 55, 60, 70, 80, 90, 100, 110 or 120 mg is administered.

According to an embodiment, a daily dose of active ingredient of desvenlafaxine ranges between 50 and 250 mg are administered. More preferably, a daily dose of 50, 75, 100, 125, 150, 175, 200, 225 or 250 mg is administered.

The mental disorders which can be treated using pipamperone in a combination therapy with a selective serotonin, nor-adrenaline and dopamine re-uptake inhibitor (SNDRI) compound, are mood disorders or anxiety disorders.

The present invention thus relates to the use of pipamperone or a pharmaceutically acceptable salt thereof for the preparation of a medicament for treating mood disorders or anxiety disorders, including depression, characterized in that pipamperone or said pharmaceutically acceptable salt thereof is administered simultaneously with, separate from or prior to the administration of an SNDRI compound to augment the therapeutic effect or to provide a faster onset of the therapeutic effect of said SNDRI, further characterized in that pipamperone is to be administered to a patient in a daily dose ranging between 15, preferably more than 15 mg, and about 20 mg of the active ingredient. According to a preferred embodiment, the invention relates to the uses as described above, wherein said SNDRI compound is selected from the group consisting of NS 2330; NS 2359, McN 5652; DOV 216,303 and DOV 21,947; more preferably NS 2359 or DOV 216,303; or a pro-drug or an active metabolite thereof; or a pharmaceutically acceptable salt thereof.

The invention also relates to a pharmaceutical composition comprising (a) pipamperone, and (b) an SNDRI compound, preferably selected from the group consisting of NS 2330; NS 2359, McN 5652; DOV 216,303 and DOV 21,947, more preferably NS 2359 or DOV 216,303, or a pro-drug or an active metabolite thereof, or a pharmaceutically acceptable salt thereof, as a combined preparation for simultaneous, separate or sequential use for treating mood or anxiety disorders. The invention also relates to a pharmaceutical composition comprising (a) pipamperone in a dose ranging between 15, preferably more than 15, and about 20 mg of the active ingredient, and (b) an SNDRI compound, preferably selected from the group consisting of NS 2330; NS2359, McN 5652; DOV 216,303 and DOV 21,947, more preferably NS 2359 or DOV 216,303, or a pro-drug or an active metabolite thereof, or a pharmaceutically acceptable salt thereof.

The mental disorders which can be treated using pipamperone in a combination therapy with an SNRI compound are chosen from the group of diseases or disorders consisting of mood disorders and anxiety disorders.

The present invention thus relates to the use of pipamperone or a pharmaceutically acceptable salt thereof for the preparation of a medicament for treating mood or anxiety disorders, including depression, characterized in that pipamperone or said pharmaceutically acceptable salt thereof is administered simultaneously with, separate from or prior to the administration of an SNRI compound to augment the therapeutic effect or to provide a faster onset of the therapeutic effect of said SNRI compound, further characterized in that pipamperone is to be administered to a patient in a daily dose ranging between 15, preferably more than 15 and about 20 mg of the active ingredient. According to a preferred embodiment, the invention relates to the uses as described above, wherein said SNRI compound is selected from the group consisting of venlafaxine, tomoxetine, tandamine, talsupram, talopram, nefazodone, milnacipran, LY 113.821, duloxetine, desvenlafaxine and amoxapine, or a pro-drug or an active metabolite thereof, or a pharmaceutically acceptable salt thereof. Even more preferably, said SNRI compound is chosen from the group consisting of venlafaxine, tomoxetine, milnacipran, duloxetine and desvenlafaxine, or a pro-drug or an active metabolite thereof, or a pharmaceutically acceptable salt thereof.

More preferably, said SNRI compound is milnacipran and is to be administered in a daily dose ranging between 50 and 200 mg of the active ingredient.

More preferably, said SNRI compound is nefazodone and is to be administered in a daily dose ranging between 100 and 600 mg of the active ingredient.

More preferably, said SNRI compound is amoxapine and is to be administered in a daily dose ranging between 100 and 600 mg of the active ingredient.

More preferably, said SNRI compound is venlafaxine and is to be administered in a daily dose ranging between 75 and 300 mg of the active ingredient.

More preferably, said SNRI compound is duloxetine and is to be administered in a daily dose ranging between 40 and 120 mg of the active ingredient.

More preferably, said SNRI compound is tomoxetine and is to be administered in a daily dose ranging between 0.475 and 3.8 mg/kg of the active ingredient.

More preferably, said SNRI compound is desvenlafaxine and is to be administered in a daily dose ranging between 50 and 250 mg of the active ingredient.

The invention also relates to a pharmaceutical composition comprising (a) pipamperone in a dose ranging between 15, preferably more than 15 and about 20 mg of the active ingredient, and (b) an SNRI compound, preferably selected from the group consisting of venlafaxine, tomoxetine, tandamine, talsupram, talopram, nefazodone, milnacipran, LY 113.821, duloxetine, desvenlafaxine and amoxapine, or a pro-drug or an active metabolite thereof, or a pharmaceutically acceptable salt thereof, as a combined preparation for simultaneous, separate or sequential use for treating mood or anxiety disorders. The invention also relates to a pharmaceutical composition comprising (a) pipamperone in a dose ranging between 15, preferably more than 15 and about 20 mg of the active ingredient, and (b) an SNRI compound, preferably selected from the group consisting of venlafaxine, tomoxetine, tandamine, talsupram, talopram, nefazodone, milnacipran, LY 113.821, duloxetine, desvenlafaxine and amoxapine, or a pro-drug or an active metabolite thereof, or a pharmaceutically acceptable salt thereof.

The invention also relates to a pharmaceutical composition as described above wherein pipamperone is provided in a unitary dose ranging between 15, preferably more than 15 and about 20 mg of the active ingredient and wherein said SNRI compound is milnacipran, preferably provided in a unitary dose of between 50 and 200 mg of the active ingredient.

The invention also relates to a pharmaceutical composition as described above wherein pipamperone is provided in a unitary dose ranging between 15, preferably more than 15 and about 20 mg of the active ingredient and wherein said SNRI compound is nefazodone, preferably provided in a unitary dose of between 100 and 600 mg of the active ingredient.

The invention also relates to a pharmaceutical composition as described above wherein pipamperone is provided in a unitary dose ranging between 15, preferably more than 15 and about 20 mg of the active ingredient and wherein said SNRI compound is amoxapine, preferably provided in a unitary dose of between 100 and 600 mg of the active ingredient.

The invention also relates to a pharmaceutical composition as described above wherein pipamperone is provided in a unitary dose ranging between 15, preferably more than 15 and about 20 mg of the active ingredient and wherein said SNRI compound is venlafaxine, preferably provided in a unitary dose of between 75 and 300 mg of the active ingredient. The invention also relates to a pharmaceutical composition as described above wherein pipamperone is provided in a unitary dose ranging between 15, preferably more than 15 and about 20 mg of the active ingredient and wherein said SNRI compound is duloxetine, preferably provided in a unitary dose of between 40 and 120 mg of the active ingredient.

The invention also relates to a pharmaceutical composition as described above wherein pipamperone is provided in a unitary dose ranging between 15, preferably more than 15 and about 20 mg of the active ingredient and wherein said SNRI compound is tomoxetine, preferably provided in a unitary dose of between 0.475 and 3.8 mg/kg of the active ingredient.

The invention also relates to a pharmaceutical composition as described above wherein pipamperone is provided in a unitary dose ranging between 15, preferably more than 15 and about 20 mg of the active ingredient and wherein said SNRI compound is desvenlafaxine, preferably provided in a unitary dose of between 50 and 250 mg of the active ingredient.

According to an embodiment, a daily dose of active ingredient of SNRI, preferably milnacipran ranges between 50 and 200 mg are administered. More preferably, a daily dose of 50, 75, 100, 125, 150, 175 or 200 mg is administered.

According to an embodiment, a daily dose of active ingredient of SNRI, preferably nefazodone ranges between 100 and 600 mg are administered. More preferably, a daily dose of 100, 150, 200, 250, 300, 350, 400, 450, 500, 550 or 600 mg is administered.

According to an embodiment, a daily dose of active ingredient of SNRI, preferably amoxapine ranges between 100 and 600 mg are administered. More preferably, a daily dose of 100, 150, 200, 250, 300, 350, 400, 450, 500, 550 or 600 mg is administered.

According to an embodiment, a daily dose of active ingredient of SNRI, preferably venlafaxine ranges between 75 and 300 are administered. More preferably, a daily dose of 75, 100, 125, 150, 175, 200, 225, 250, 275 or 300 mg is administered.

According to an embodiment, a daily dose of active ingredient of SNRI, preferably duloxetine ranges between 40 and 120 mg are administered. More preferably, a daily dose of 40, 45, 50, 55, 60, 70, 80, 90, 100, 110 or 120 mg is administered.

According to an embodiment, a daily dose of active ingredient of SNRI, preferably tomoxetine ranges between 0.475 and 3.8 mg/kg are administered. More preferably, a daily dose of 0.475, 0.95, 1.0, 1.2, 1.5, 1.75, 1.9, 2.0, 2.25, 2.5, 2.75, 3.0, 3.5 or 3.8 mg/kg is administered.

According to an embodiment, a daily dose of active ingredient of SNRI, preferably desvenlafaxine ranges between 50 and 250 mg are administered. More preferably, a daily dose of 50, 75, 100, 125, 150, 175, 200, 225 or 250 mg is administered.

The invention, now being generally described, will be more readily understood by reference to the following tables and examples, which are included merely for purposes of illustration of certain aspects and embodiments of the present invention and are not intended to limit the invention.

### TABLES: Short description

| | |
|---|---|
| **Table 1:** | pKi values of test compounds are given for each of the dopamine receptors, 5HT receptors, adrenergic receptors and the histamine1 receptor. |
| **Table 2:** | Set-up of a clinical trial comprising for treatment groups. |
| **Table 3:** | Overview SSRI, SNDRI and SNRI compounds. |

### EXAMPLES

### EXAMPLE 1: Foregoing pipamperone-citalopram treatment in major depressive disorder: a placebo and active controlled period finding clinical trial

Table 2 represents the set-up of a clinical trial comprising for treatment groups:
Group Plc - Active / Day 0 represents the group receiving 10 mg citalopram, twice a day, starting the first day (Day 0) of active treatment in the clinical trial. This administration regime is also indicated as the mono therapy.
Group Pip - Active / Day 0 represents the group receiving a combination of 8.5 mg pipamperone and 10 mg citalopram, twice a day, starting the first day (Day 0) of active treatment in the clinical trial. This administration regime is also indicated as the non-foregoing combo therapy.
Group Pip - Active / Day 4 represents the group receiving 8.5 mg pipamperone, twice a day, starting the first day (Day 0) of active treatment in the clinical trial, followed by a combination of 8.5 mg pipamperone and 10 mg citalopram, twice a day, starting the fifth (Day 4) day of active treatment in the clinical trial. This administration regime is also indicated as the foregoing therapy with combination therapy starting after 4 days of active treatment.
Group Pip - Active / Day 7 represents the group receiving 8.5 mg pipamperon, twice a day, starting the first day (Day 0) of active treatment in the clinical trial, followed by a combination of 8.5 mg pipamperone and 10 mg citalopram, twice a day, starting the eight (Day 7) day of active treatment in the clinical trial. This administration regime is also indicated as the foregoing therapy with combination therapy starting after 7 days of active treatment.

All subjects also undergo a placebo (PLC) run-in therapy, administered during a period of about 7 days before the active treatment starts.

During daily (D), weekly (W) or monthly (M) visits, several parameters are measured.

Under NECT is to be understood: Neuronal E-clinical Trial = Vesalius Expert development for this trial which includes the bottom-up measurement of:
- In- and exclusion-criteria
- Functional status evaluation
- Medical history
- (Pre-)treatment signs & symptoms
- DSM-IV rules for diagnosis & efficacy
- HDRS-28 (Hamilton Depression Rating Scale - 28 items)
- Medical resource utilisation
- Pre-trial & Concomitant medication
- Drug administration
- (Serious) Adverse events
- Admission to the acute and extension phase of treatment
- Right flow of the trial

### EXAMPLE 2: combo pipamperone-citalopram: therapeutic use in Major Depression

### Purpose

Pipamperone, administered to patients in a dose ranging between 16 and 19 mg is claimed via its specific pharmacological properties to be a booster of the antidepressant effect of the selective serotonin re-uptake inhibitor citalopram. The mechanism of boosting of pipamperone has to deal with (i) the selective affinity for the dopamine-4 (D4) receptor with a pKi value equal to or higher than 8 towards the D4 receptor and less than 8 towards other dopamine receptors, and (ii) the selective affinity for the 5-HT2A receptor with a pKi value equal to or higher than 8 towards the 5-HT2A receptor and less than 8 towards other 5HT receptors. This semi-naturalistic open label study investigated the efficacy and tolerability of the combo pipamperone - citalopram in the treatment of patients with major depression.

### Details

Design: Semi-naturalistic i.e. inclusion of every 'natural' patient in an outpatient practice but without concomitant use of mood enhancing drugs, open label
Control: No
Phase: Phase IIa - preliminary Proof of Concept
Location: Belgium - Research Centre ANIMA, Alken
End Points : Assessment scale scores, Hamilton Depression Rating Scale 17 items, Reduction, Response, Remission
Medication: Exclusion of mood stabilisers, antipsychotics (typical and atypical) and other antidepressants

### Subjects

Patients have a major depressive disorder according to DSM-IV criteria, with or without a chronic course and a treatment refractory state towards another SSRI than citalopram.

### Treatments

PIP-CIT¹ add-on: citalopram from day minus 60-20 - pipamperone from DAY 0

| **Drug/Treatment** | **Dose (total)** | **Route** | **Duration** |
|---|---|---|---|
| Pipamperone¹ + | Pip.: 16-19 mg/day | PO | 8 weeks |
| Citalopram¹ | Cit.: 10-20 mg/day | | |

| | | | |
|---|---|---|---|
| 1. Pipamperone (Pip) and citalopram (Cit) dosage was adjusted according to clinical response. PIP-CIT¹ fore-going 1-5: pipamperone from day 0 - citalopram from day 1-5 | | | |

| **Drug/Treatment** | **Dose (total)** | **Route** | **Duration** |
|---|---|---|---|
| Pipamperon¹ + | Pip.: 16-19 mg/day - | PO | 8 weeks |
| Citalopram¹ | Cit.: 10-20 mg/day | | |

| | | | |
|---|---|---|---|
| 1. Pipamperone (Pip) and citalopram (Cit) dosage was adjusted according to clinical response. PIP-CIT¹ fore-going 6-8: pipamperone from day 0 - citalopram from day 6-8 | | | |

| **Drug/Treatment** | **Dose (total)** | **Route** | **Duration** |
|---|---|---|---|
| Pipamperon¹ + | Pip: 16-19 mg/day | PO | 8 weeks |
| Citalopram¹ | Cit: 10-20 mg/day | | |

| | | | |
|---|---|---|---|
| 1. Pipamperone (Pip) and citalopram (Cit) dosage was adjusted according to clinical response. | | | |

### Results

The results for the add-on, foregoing 1-5 and foregoing 6-8 PIP-CIT are compared with:
(1) standard efficacy of antidepressants in clinical trials according to Khan et al. (2000), in "Symptom Reduction and Suicide Risk in Patients Treated With Placebo in Antidepressant Clinical Trials" (Arch. of General Psychiatry, Vol. 57, April 2000).
(2) HDRS-17 change from baseline vs SNRI (duloxetine) in Major Depression; the SNRI (duloxetine) treatment was 40-120 mg/day (n = 152) according to Goldstein *et al.,* (Clin. Psychiatry).
(3) remission rates (HDRS-17 <=7) vs SSRIs vs placebo in Major Depression; treatment with SSRIs is according to a meta-analysis of Thase et al. (Br. J. Psychiatry (2001) 178:234-241). Treatment with placebo is according to a meta-analysis of Thase et al. (Br. J. Psychiatry (2001) 178:234-241).
(4) W02005/053796

### Adverse Events

Adverse events are assessed on: body as a whole, central and peripheral nervous system, gastrointestinal, musculoskeletal, psychiatric, respiratory, skin and appendages, vascular and urinary, taking into account discontinued treatment due to adverse events. Laboratory parameters, ECG, bodyweight and vital signs are not measured since this is a naturalistic study.

### Study Messages

The boosting effect of pipamperone at an unconventional low dose on a SSRI, in this case citalopram, is apparent.

The combination pipamperone-citalopram is generally well tolerated in patients with depression i.e. at least no specific added adverse events are expected by adding pipamperone at the doses used in the study.

Sedative effects of pipamperone, which are observed at higher doses, are lacking.

### EXAMPLE 3: POC process for major depressive disorder

### Concept:

Combo of the high selective 5-HT2A/D4 antagonist pipamperone with:
- citalopram and/or escitalopram;
- a fore-going admission during 4 days of pipamperone;
- a dose of pipamperone of 17 mg/day

### Objectives:

Demonstrating that this combo therapy has:
- the potency of being a treatment standard for depression by having an added value of reducing the total score of the Hamilton Depression Rating Scale-17 items (HDRS-17) after 8 weeks of therapy with a least 20% more than reached with the conventional known antidepressants, i.e. 60% versus 40%. This stands for an added medium demission of 5 points on the total score of the HDRS-17 and by this will be very highly significant since the mean difference in all recent clinical trials between placebo and active treatment is 2.5;
- a more sustained therapeutic effect than the conventional mono therapy by preventing significant more relapses during 48 weeks following the acute treatment; and/or
- a complete neutral safety profile, e.g. there are no more adverse events in the combo therapy then in mono admission of the in the combo used antidepressant compound.

### Process:

The following different steps were implemented to reach out for these objectives (see also Table 2):
(1) an naturalistic open label study (n=>20) on a depressive population with a normal variability of medical and psychiatric history, course of depression, earlier and concomitant therapy admitting the golden standard antidepressant citalopram 20-40 mg/day and a dose of 16-19 mg/day of pipamperone in a foregoing, simultaneous or add-on use.
(2) a 16 weeks placebo controlled randomised four armed study of each 36 patients with a mayor depressive disorder admitting:
   - from day 0: pipamperone (PIP) 17 mg/day or an active antidepressant compound or the combination of the last two;
   - from day 4: pipamperone 17 mg/day combined with and without an active antidepressant compound or an active antidepressant compound without pipamperone;
   - from day 7: pipamperone 17 mg/day combined with an active antidepressant compound or an active antidepressant compound without pipamperone.

By including rigorous control groups (placebo and active comparator; see Table 2) this clinical trial is evaluated as a proof of concept of the added value of the combo and the foregoing treatment method since the inclusion/exclusion of:
- a negative trial, i.e. no significant difference between the placebo and active treatment with the comparator;
- a failed trial, i.e. no significant difference between the active and the studied treatment i.e. the combo.

(3) an active controlled randomised relapse prevention study following the POC trial during another 36 weeks with three arms of each 36 patients which is formed by:
   - continuation of the active mono therapy;
   - randomising the patients with a combo therapy in a group with an active mono therapy and with a continuation of the combo treatment.

### EXAMPLE 4: combo pipamperone-citalopram: therapeutic use in Obsessive-Compulsive Disorder (OCD).

### Purpose

Pipamperone (1'-[3-(p-Fluorobenzoyl)propyl]-[1,4'-bipiperidine]-4'-carboxamide), the active ingredient of Dipiperon (Janssen-Cilag B.V), is administered to a patient in a dose ranging between 16 and 19 mg, is claimed via its specific pharmacological properties to be a booster of the effect of the selective serotonin re-uptake inhibitor citalopram towards OCD. The mechanism of boosting of pipamperone has to deal with (i) the selective affinity for the dopamine-4 (D4) receptor with a pKi value equal to or higher than 8 towards the D4 receptor and less than 8 towards other Dopamine receptors, and (ii) the selective affinity for the 5-HT2A receptor with a pKi value equal to or higher than 8 towards the 5-HT2A receptor and less than 8 towards other 5HT receptors. This semi-naturalistic open label study investigates the efficacy and tolerability of the combo pipamperone - citalopram in the treatment of patients with OCD.

### Details

| | |
|---|---|
| Design: | Semi-naturalistic i.e. inclusion of every 'natural' patient in an outpatient practice but without concomitant use of anxiolytics, open label |
| Control: | No |
| Phase: | Phase Ila - preliminary Proof of Concept |
| Location: | Belgium - Research Centre ANIMA, Alken |
| End Points : | Assessment scale scores, Yale-Brown Obsessive-Compulsive Scale, Reduction, Remission |
| Medication: | Exclusion of mood stabilisers, antipsychotics (typical and atypical) and other antidepressants and anxiolytics |

### Subjects

Characteristics: patients have an obsessive-compulsive disorder according to DSM-IV criteria, with or without a chronic course and a treatment refractory state towards another SSRI than citalopram.

### Treatments

PIP-CIT ADD-ON: citalopram from DAY minus 730-60 - pipamperone from DAY 0, in which Pipamperone (Pip) and Citalopram (Cit) dosage is adjusted according to clinical response.

PIP-CIT FORE-GOING 4-6: pipamperone from DAY 0 - citalopram from DAY 4-6, in which Pipamperone (Pip) and Citalopram (Cit) dosage is adjusted according to clinical response.

### Results

The results are scored via Y-BOCS total score: "fore-going" and "add-on" treatment with pipamperone (16-19 mg/day) and citalopram (30-60 mg/day; bid) in comparison with the SSRI fluvoxamine in OCD. Treatment with the combo pipamperone-citalopram (n = 7). Treatment with fluvoxamine (controlled release) mean 271 mg/day (n = 253) is according to Hollander et al. (2003).

The intention-to-treat/last-observation-carried-forward analysis is evaluated relating to therapeutic efficacy according Y-BOCS total score, obsession and compulsion scores.

### Assessment

Efficacy: the combo pipamperone 16-19 mg/day - citalopram 30-60 mg/day was assessed with the in the art known compounds effective towards OCD (Hollander E, Koran LM, Goodman WK, Greist JH, Ninan PT, et al. A double-blind, placebo-controlled study of the efficacy and safety of controlled-release fluvoxamine in patients with obsessive-compulsive disorder. Journal of Clinical Psychiatry 64: 640-647, June 2003 Mount Sinai School of Medicine, New York, New York, USA; Solvay Pharmaceuticals Inc., Marietta, Georgia, USA).

### Study Messages

The boosting effect of pipamperone at an unconventional low dose on a SSRI is studied relating to the efficacy of the 'add-on' and 'fore-going' combo 'pipamperone 16-19 mg/day - citalopram 30-60 mg/day' in the treatment of patients with obsessive-compulsive disorder.

The combination pipamperone-citalopram is assessed in patients with OCD, no specific added adverse events are expected by adding pipamperone at the doses used in the study.

Sedative effects of pipamperone, which are observed at higher doses, are lacking.

**EXAMPLE 5: combo pipamperone-citalopram: therapeutic use in Panic Disorder.**

Purpose Preliminary examination of a "fore-going" and "add-on" treatment with pipamperone (16-19 mg/day) and citalopram (20-60 mg/day) in comparison with the SSRI in Panic Disorder.

### Subjects

Characteristics: patients have Panic Disorder according to DSM-IV criteria.

### Treatments

PIP-CIT ADD-ON: citalopram from DAY minus 24-2 months - pipamperone from DAY 0, in which Pipamperone (Pip) and Citalopram (Cit) dosage is adjusted according to clinical response.

PIP-CIT FORE-GOING 4-6: pipamperone from DAY 0 - citalopram from DAY 4-6, in which Pipamperone (Pip) and Citalopram (Cit) dosage is adjusted according to clinical response.

### Details

| | |
|---|---|
| Design: | Semi-naturalistic i.e. inclusion of every 'natural' patient in an outpatient practice but without concomitant use of anxiolytics, open label |
| Control: | No |
| Phase: | Phase Ila - preliminary Proof of Concept |
| Location: | Belgium - Research Centre ANIMA, Alken |
| End Points : | Assessment scale scores, CGI-severity score, Reduction, Remission |
| Medication: | Exclusion of mood stabilisers, antipsychotics (typical and atypical) and other antidepressants and anxiolytics |

### Results

The results are depicted by the CGI-severity score: "fore-going" and "add-on" treatment with pipamperone (16-19 mg/day) and citalopram (20-60 mg/day) in comparison with the SSRI in Panic Disorder. Treatment with the combo pipamperone-citalopram. Treatment with paroxetine is according to the Journal of Clinical Psychiatry (2004) 65: 405-413. Treatment with Sertraline is according to the Journal of Clinical Psychiatry (2004) 65: 405-413.

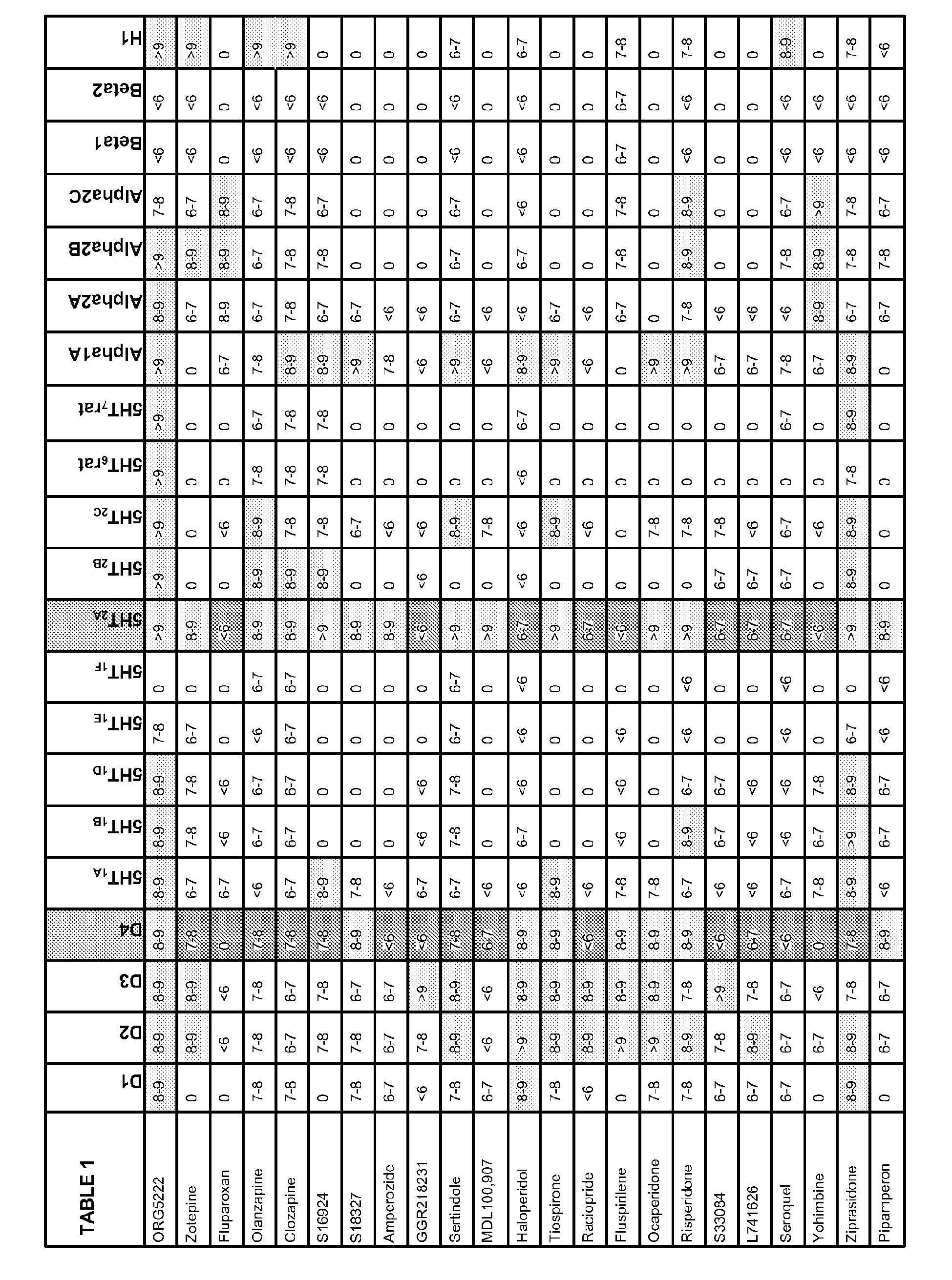

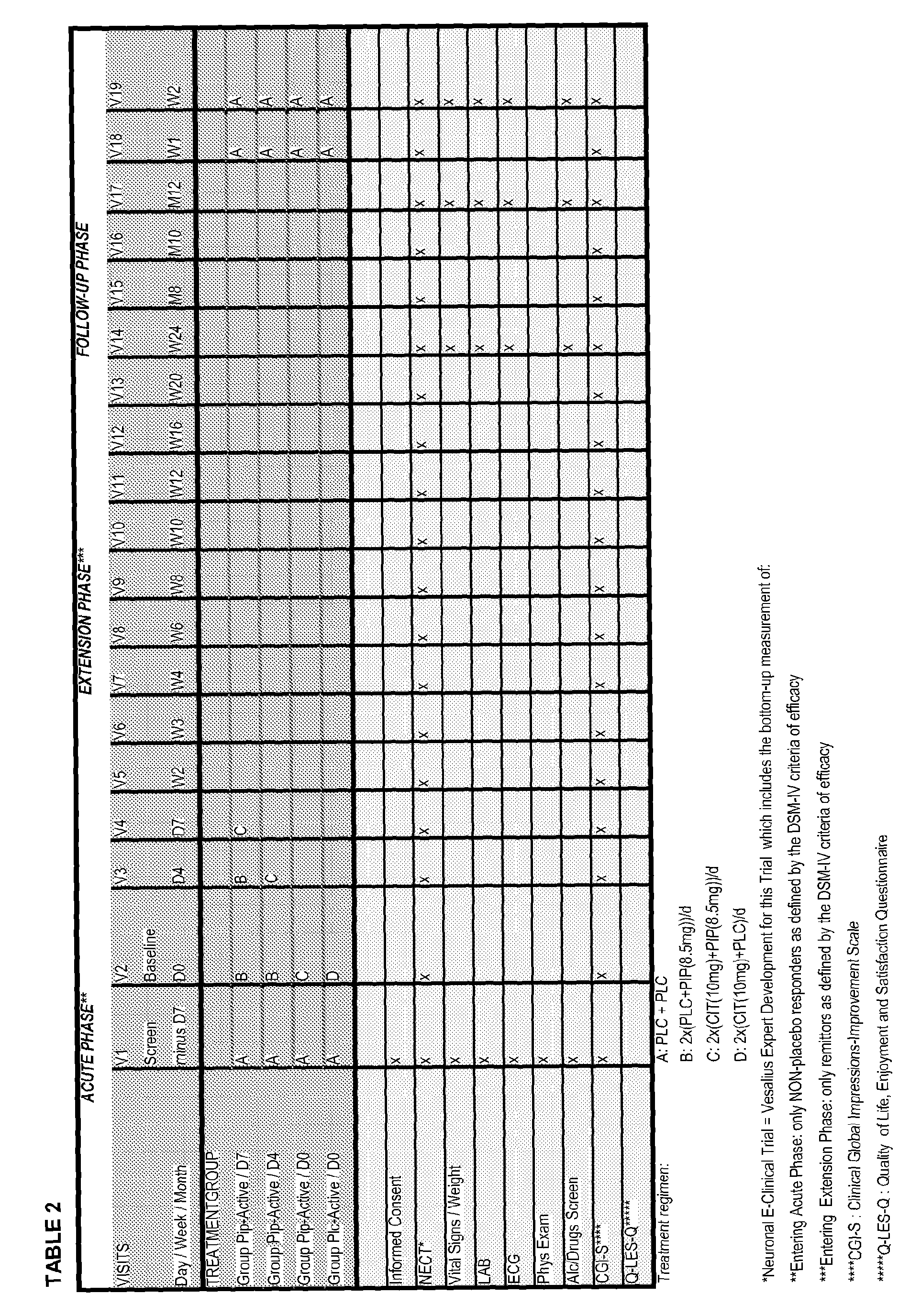

| PHARMAC. GROUP | PHARMACOLOGICAL PROFILE | MAIN INDICATIONS | COMPOUND | DOSE RANGE | COMPANY | |
|---|---|---|---|---|---|---|
| Monoaminergic Transmitter Systems | SNDRI | Alzheimer's Disease / Depression | NS 2330 | not available | Boehringer-Ingelheim Pharmaceuticals | |
| | SNDRI | Alzheimer's Disease / Depressior | NS 2359 | not available | Boehringer Ingelheim Pharmaceuticals | |
| | SNDRI | Depression / Anxiety | DOV 216,303 | not available | DOV | |
| | SNDRI | Alzheimer's Disease / Depression | DOV 21,947 | not available | DOV | |
| | SNDRI | Depression | DOV 21,947 | not available | DOV | |
| | SNDRI | Depression | MoN 5652 | not available | MoNeil | |
| Monoaminergic Transmitter Systems | SNRI | Depression | milnacipran | 50 to 200mg daily | Pierre Fabre | |
| | SNRI | Depression / Anxiety | nefazodone | 100 to 600 mg daily | Mead Johnson | |
| | SNRI | Depression / Anxiety | amoxapine | 100 to 600 mg daily | Weyth | |
| | SNRI | Depression / Anxiety | venlafaxine | 75 to 300mg daily | Wyeth | |
| | SNRI | Depression / Anxiety | duloxetine | 40 to 120 mg daily | Eli Lilly | |
| | SNRI | ADHD / Depression | tomoxetine | 1.9mg/kg/day | Lilly | |
| | SNRI | Depression / Anxiety | desvenlafaxine | 50 to 250 mg daily | Wyeth | |
| | SNRI | Depression | talsupram | not available | Lundbeck | |
| | SNRI | Depression | talopram | not available | Lundbeck / Weyth | |
| | SNRI | Depression | tandamine | not available | Wyeth | |
| | SNRI | Depression | LY 113.821 | not available | Lilly | |
| Monoaminergic Transmitter Systems | SSRI | Depression / Anxiety | paroxetine | 20 75 mg daily | GlaxoSmithKline | |
| | SSRI | Depression / Anxiety | escitalopram | 5 to 50 mg daily | Lundbeck / Forest Laboratories | |
| | SSRI | Depression / Anxiety | citalopram | 5 to 60 mg daily | off parent | |
| | SSRI | Depression / Anxiety | fluoxetine | 20 60 mg daily | off parent | |
| | SSRI | Depression / Anxiety | fluvoxamine | 50 300 mg daily | off parent | |
| | SSRI | Depression / Anxiety | semraline | 25 300 mg daily | Pfizer | |
| | SSRI | Anxiety (OCD / Soc Phobia) | fluvoxamine controlled release | 100 to 300mg daily | Solvay | |
| | SSRI | Depression / Anxiety | litoxetine | not available | Sanofi Synthelabo | |
| | SSRI | Depression / Anxiety | femoxetine | not available | Ferrosan | |
| | SSRI | Depression / Anxiety | ifoxetine | not available | Novartis Pharmaceuticals | |
| | SSRI | Depression | VPI 013 (also known as OPC 14523) | not available | Vela, Otsuka | |
| | SSRI | Depression / Anxiety | EMD 68843 | not available | EMD Phamaceulicals | |
| | SSRI | Depression / Anxiety | ericlamine | not available | Jouveinal | |
| | SSRI | Depression | Lu 35-138 | not available | Lundbeck | |
| | SSRI | Depression / OCD / Pain | LY 214.281 | not available | Lilly | |
| | SSRI | Depression | LU AA 21-004 | not available | Lundbeck | |
| | SSRI | Depression / Anxiety | cyanodothepine | not available | ? | |
| | SSRI | Depression / Anxiety | ademethionine / s adenosylmethionine ne | not available | Sampl Gibipharma | |
| | SSRI | Depression / Anxiety | YM 992 | not available | Yamanouchi | |
| | SSRI | Depression | milnacipran | 50 to 200mg daily | Pierre Fabre | |
| | SSRI | Depression / Anxiety | venlafaxine | 75 to 300mg daily | Wyeth | |
| | SSRI | Depression / Anxiety | duloxetine | 40 to 120 mg daily | Eli Lilly | |
| | SSRI | Depression / Anxiety | desvenlafaxine | 50 to 250 mg daily | Wyeth | |

## Claims

1. Use of pipamperone or a pharmaceutically acceptable salt thereof for the preparation of a medicament for treating a mood disorder or anxiety disorder, wherein said pipamperone or a pharmaceutically acceptable salt is to be administered to a patient in a daily dose ranging between more than 15 and about 20 mg of the active ingredient, and wherein a second compound is to be administered simultaneously with, separate from or sequential to said pipamperone to augment the therapeutic effect of said second compound or to provide a faster onset of the therapeutic effect of said second compound, said second compound is selected from the group consisting of: selective serotonin, nor-adrenaline and dopamine re-uptake inhibitor (SNDRI), selective serotonin and nor-adrenaline re-uptake inhibitor (SNRI) and selective serotonin re-uptake inhibitor (SSRI).

2. The use according to claim 1, wherein said pipamperone is to be administered daily at least one day before administering said second compound.

3. The use of pipamperone or a pharmaceutically acceptable salt thereof according to any of claims 1 to 2, wherein said second compound is a selective serotonin, nor-adrenaline and dopamine re-uptake inhibitor (SNDRI) compound.

4. The use according to claim 3, wherein said selective serotonin, nor-adrenaline and dopamine re-uptake inhibitor (SNDRI) compound is selected from the group consisting of NS 2330, NS 2359, McN 5652, DOV 216,303 and DOV 21,947, or a pharmaceutically acceptable salt thereof.

5. A pharmaceutical composition comprising (a) pipamperone, and (b) a selective serotonin, nor-adrenaline and dopamine re-uptake inhibitor (SNDRI) compound, for treating a mood disorder or anxiety disorder, wherein said pipamperone is to be administered to a patient in a daily dose ranging between more than 15 and about 20 mg of the active ingredient.

6. The pharmaceutical composition according to claim 5, wherein said selective serotonin, nor-adrenaline and dopamine re-uptake inhibitor (SNDRI) compound is selected from the group consisting of NS 2330, NS 2359, McN 5652, DOV 216,303 and DOV 21,947, or a pharmaceutically acceptable salt thereof, preferably NS 2359.

7. The use of pipamperone or a pharmaceutically acceptable salt thereof according to any of claims 1 to 2, wherein said second compound is a selective serotonin and nor-adrenaline re-uptake inhibitor (SNRI) compound.

8. The use of pipamperone or a pharmaceutically acceptable salt thereof according to claim 7, wherein said selective serotonin and nor-adrenaline re-uptake inhibitor (SNRI) compound is selected from the group consisting of venlafaxine, tomoxetine, tandamine, talsupram, talopram, nefazodone, milnacipran, LY 113.821, duloxetine, desvenlafaxine and amoxapine, or a pharmaceutically acceptable salt thereof.

9. The use of pipamperone or a pharmaceutically acceptable salt thereof according to claim 8, wherein said
- venlafaxine, or a pharmaceutically acceptable salt thereof, is to be administered in a daily dose ranging between 75 and 300 mg of the active ingredient;
- tomoxetine, or a pharmaceutically acceptable salt thereof, is to be administered in a daily dose ranging between 0.475 and 3.8 mg/kg of the active ingredient;
- milnacipran, or a pharmaceutically acceptable salt thereof, is to be administered in a daily dose ranging between 50 and 200 mg of the active ingredient;
- duloxetine, or a pharmaceutically acceptable salt thereof, is to be administered in a daily dose ranging between 40 and 120 mg of the active ingredient;
- nefazodone, or a pharmaceutically acceptable salt thereof, is to be administered in a daily dose ranging between 100 and 600 mg of the active ingredient;
- amoxapine, or a pharmaceutically acceptable salt thereof, is to be administered in a daily dose ranging between 100 and 600 mg of the active ingredient; and/or
- desvenlafaxine, or a pharmaceutically acceptable salt thereof, is to be administered in a daily dose ranging between 50 and 250 mg of the active ingredient.

10. A pharmaceutical composition comprising (a) pipamperone and (b) a selective serotonin and nor-adrenaline re-uptake inhibitor (SNRI) compound for treating a mood disorder or anxiety disorder, wherein said pipamperone is to be administered to a patient in a daily dose ranging between more than 15 and about 20 mg of the active ingredient.

11. The pharmaceutical composition according to claim 10, wherein said selective serotonin and nor-adrenaline re-uptake inhibitor (SNRI) compound is selected from the group consisting of venlafaxine, tomoxetine, tandamine, talsupram, talopram, nefazodone, milnacipran, LY 113.821, duloxetine, desvenlafaxine and amoxapine, or a pharmaceutically acceptable salt thereof,

12. The pharmaceutical composition according to claim 11, wherein said
- venlafaxine, or a pharmaceutically acceptable salt thereof, provided in a unitary dose of between 75 and 300 mg of the active ingredient;
- tomoxetine, or a pharmaceutically acceptable salt thereof, is provided in a unitary dose of between 0.475 and 3.8 mg/kg of the active ingredient;
- milnacipran, or a pharmaceutically acceptable salt thereof, is provided in a unitary dose of between 50 and 200 mg of the active ingredient;
- duloxetine, or a pharmaceutically acceptable salt thereof, is provided in a unitary dose of between 40 and 120 mg of the active ingredient;
- nefazodone, or a pharmaceutically acceptable salt thereof, is provided in a unitary dose of between 100 and 600 mg of the active ingredient;
- amoxapine, or a pharmaceutically acceptable salt thereof, is provided in a unitary dose of between 100 and 600 mg of the active ingredient; and/or
- desvenlafaxine, or a pharmaceutically acceptable salt thereof, is provided in a unitary dose of between 50 and 250 mg of the active ingredient.

13. The use of pipamperone or a pharmaceutically acceptable salt thereof according to any of claims 1 to 2, wherein said second compound is a selective serotonin re-uptake inhibitor (SSRI) compound.

14. The use of pipamperone or a pharmaceutically acceptable salt thereof according to claim 13, wherein said selective serotonin re-uptake inhibitor (SSRI) compound is selected from the group consisting of YM 992, VPI-013 (OPC-14523), sertraline, paroxetine, LY 214.281, LU AA 21-004, Lu 35-138, litoxetine, ifoxetine, fluvoxamine (controlled release formulation), fluvoxamine, fluoxetine, femoxetine, escitalopram, EMD 68843, cyanodothepine, citalopram, venlafaxine, milnacipran, duloxetine, desvenlafaxine cericlamine and ademethionine, or a pharmaceutically acceptable salt thereof.

15. The use of pipamperone or a pharmaceutically acceptable salt thereof according to claim 14, wherein said:
- fluvoxamine (controlled release formulation), or a pharmaceutically acceptable salt thereof, is to be administered in a daily dose ranging between 100 and 300 mg of the active ingredient;
- escitalopram, and is to be administered in a daily dose ranging between 5 and 50 mg of the active ingredient;
- citalopram, and is to be administered in a daily dose ranging between 5 and 60 mg of the active ingredient;
- paroxetine and is to be administered in a daily dose ranging between 20 and 75 mg of the active ingredient;
- fluoxetine and is to be administered in a daily dose ranging between 20 and 60 mg of the active ingredient;
- fluvoxamine and is to be administered in a daily dose ranging between 50 and 300 mg of the active ingredient;
- sertraline and is to be administered in a daily dose ranging between 25 and 300 mg of the active ingredient;
- milnacipran and is to be administered in a daily dose ranging between 50 and 200 mg of the active ingredient;
- venlafaxine and is to be administered in a daily dose ranging between 75 and 300 mg of the active ingredient;
- duloxetine and is to be administered in a daily dose ranging between 40 and 120 mg of the active ingredient; and/or
- desvenlafaxine and is to be administered in a daily dose ranging between 50 and 250 mg of the active ingredient.

16. A pharmaceutical composition comprising (a) pipamperone and (b) a selective serotonin re-uptake inhibitor (SSRI) compound for treating a mood disorder or anxiety disorder, wherein said pipamperone is to be administered to a patient in a daily dose ranging between more than 15 and about 20 mg of the active ingredient.

17. The pharmaceutical composition according to claim 16, wherein said selective serotonin re-uptake inhibitor (SSRI) compound is selected from the group consisting of YM 992, VPI-013 (also known as OPC-14523), sertraline, paroxetine, LY 214.281, LU AA 21-004, Lu 35-138, litoxetine, ifoxetine, fluvoxamine (controlled release formulation), fluvoxamine, fluoxetine, femoxetine, escitalopram, EMD 68843, cyanodothepine, citalopram, venlafaxine, milnacipran, duloxetine, desvenlafaxine, cericlamine and ademethionine (preferably s-adenosylmethionine), or a pharmaceutically acceptable salt thereof.

18. The pharmaceutical composition according to claim 17, wherein said
- fluvoxamine (controlled release formulation), or a pharmaceutically acceptable salt thereof, is provided in a unitary dose of between 100 and 300 mg of the active ingredient;
- escitalopram, or a pharmaceutically acceptable salt thereof, is provided in a unitary dose of between 5 and 50 mg of the active ingredient;
- citalopram, or a pharmaceutically acceptable salt thereof, is provided in a unitary dose of between 5 and 60 mg of the active ingredient;
- paroxetine, or a pharmaceutically acceptable salt thereof, is provided in a unitary dose of between 20 and 75 mg of the active ingredient;
- fluoxetine, or a pharmaceutically acceptable salt thereof, is provided in a unitary dose of between 20 and 60 mg of the active ingredient;
- fluvoxamine, or a pharmaceutically acceptable salt thereof, is provided in a unitary dose of between 50 and 300 mg of the active ingredient;
- sertraline, or a pharmaceutically acceptable salt thereof, is provided in a unitary dose of between 25 and 300 mg of the active ingredient;
- milnacipran, or a pharmaceutically acceptable salt thereof, is provided in a unitary dose of between 50 and 200 mg of the active ingredient;
- venlafaxine, or a pharmaceutically acceptable salt thereof, is provided in a unitary dose of between 75 and 300 mg of the active ingredient;
- duloxetine, or a pharmaceutically acceptable salt thereof, is provided in a unitary dose of between 40 and 120 mg of the active ingredient; and/or
- desvenlafaxine, or a pharmaceutically acceptable salt thereof, is provided in a unitary dose of between 50 and 250 mg of the active ingredient.

19. A pharmaceutical composition comprising (a) pipamperone and (b) selective serotonin, nor-adrenaline and dopamine re-uptake inhibitor (SNDRI), selective serotonin and nor-adrenaline re-uptake inhibitor (SNRI) or selective serotonin re-uptake inhibitor (SSRI) for treating a mood disorder or anxiety disorder, said pipamperone is to be administered to a patient in a daily dose of between more than 15 and about 20 mg of the active ingredient.

20. A pharmaceutical composition comprising (a) pipamperone at between more than 15 mg and about 20 mg, and (b) selective serotonin, nor-adrenaline and dopamine re-uptake inhibitor (SNDRI), selective serotonin and nor-adrenaline re-uptake inhibitor (SNRI) or selective serotonin re-uptake inhibitor (SSRI).

21. The pharmaceutical composition according to claim 20, wherein said SNRI is selected from the group consisting of venlafaxine, tomoxetine, tandamine, talsupram, talopram, nefazodone, milnacipran, LY 113.821, duloxetine, desvenlafaxine and amoxapine, or a pharmaceutically acceptable salt thereof.

22. The pharmaceutical composition according to claim 20, wherein said SSRI is selected from the group consisting of YM 992, VPI-013 (also known as OPC-14523), sertraline, paroxetine, LY 214.281, LU AA 21-004, Lu 35-138, litoxetine, ifoxetine, fluvoxamine (controlled release formulation), fluvoxamine, fluoxetine, femoxetine, escitalopram, EMD 68843, cyanodothepine, citalopram, venlafaxine, milnacipran, duloxetine, desvenlafaxine, cericlamine and ademethionine (preferably s-adenosylmethionine), or a pharmaceutically acceptable salt thereof.

23. The pharmaceutical composition according to claim 20, wherein said SNDRI compound is selected from the group consisting of NS 2330, NS 2359, McN 5652, DOV 216,303 and DOV 21,947, or a pharmaceutically acceptable salt thereof

24. Tablets comprising (a) more than 15 and less than about 20 mg pipamperone, (b) an SNDRI, SNRI or SSRI, (c) and optionally lactose, corn starch, saccharose, talc, and/or magnesium-stearate.
